# EUROPEAN PATENT SPECIFICATION

(11) **EP 4 329 619 B1**
(45) Date of publication and mention of the grant of the patent: **17.09.2025**
(21) Application number: 22869287.7
(22) Date of filing: 14.09.2022
(51) Int. Cl.: A61B 6/00, G06T 11/00

(54) **METHODS AND SYSTEMS FOR VASCULAR ANALYSIS**
VERFAHREN UND SYSTEME ZUR GEFÄSSANALYSE
PROCÉDÉS ET SYSTÈMES D'ANALYSE VASCULAIRE

(30) Priority: 14.09.2021 CN 202111076801; 14.09.2021 CN 202111075750
(43) Date of publication of application: 06.03.2024
(73) Proprietor: Shanghai United Imaging Healthcare Co., Ltd., Shanghai 201807 (CN)
(72) Inventor: ZHAO, Xiaofen, Shanghai 201807 (CN); LI, Yang, Shanghai 201807 (CN); GUO, Huiwen, Shanghai 201807 (CN)
(74) Representative: Wang, Bo
(86) International application number: PCT/CN2022/118843
(87) International publication number: WO 2023/040918

(56) References cited:
- WO-A1-2017/148332
- AU-A1- 2004 255 014
- CN-A- 109 633 503
- CN-A- 110 728 730
- CN-A- 111 145 285
- CN-A- 112 330 800
- CN-A- 113 269 845
- CN-A- 113 616 226
- US-A1- 2021 128 095
- US-A1- 2021 153 830
- BENNINK EDWIN ET AL: "Influence of Thin Slice Reconstruction on CT Brain Perfusion Analysis", vol. 10, no. 9, 11 September 2015 (2015-09-11), US, pages e0137766, XP093143529, ISSN: 1932-6203, Retrieved from the Internet <URL:https://journals.plos.org/plosone/article/file?id=10.1371/journal.pone.0137766&type=printable> DOI: 10.1371/journal.pone.0137766
- ANDREAS FIESELMANN ET AL: "Deconvolution-Based CT and MR Brain Perfusion Measurement: Theoretical Model Revisited and Practical Implementation Details", INTERNATIONAL JOURNAL OF BIOMEDICAL IMAGING, vol. 26, no. 6, 31 January 2011 (2011-01-31), pages 1 - 20, XP055128513, ISSN: 1687-4188, DOI: 10.1155/2011/467563
- KONSTAS A.A. ET AL: "Theoretic Basis and Technical Implementations of CT Perfusion in Acute Ischemic Stroke, Part 2: Technical Implementations", vol. 30, no. 5, 1 May 2009 (2009-05-01), US, pages 885 - 892, XP093143361, ISSN: 0195-6108, Retrieved from the Internet <URL:https://www.ajnr.org/content/ajnr/30/5/885.full.pdf> DOI: 10.3174/ajnr.A1492

## Description

### TECHNICAL FIELD

The present disclosure relates to a technology field of scanning diagnosis, in particular, relates to systems and methods for vascular analysis.

### BACKGROUND

Computed Tomography (CT) perfusion imaging reflects a blood supply of tissues and organs, compared that an ordinary CT plain scanning and enhanced scanning only acquire data information of a single point, the perfusion imaging may obtain a Time Attenuation Curve (TAC) of each element of the tissues or organs by continuously scanning a plurality of phases, which can reflect an inflow and outflow stage (i.e., a blood perfusion) of a contrast agent in the tissues. Further, different mathematical models may be used to calculate various perfusion parameters (e.g., a cerebral blood volume (CBF), a local cerebral blood volume (CBV), a mean transit time (MTT), a time to peak (TTP), etc.), to form a perfusion parameter map, evaluate tissue ischemia, and guide a formulation of a treatment plan.

With the emergence of wide-body detectors and dynamic cradle technology, current perfusion scanning can realize one-stop cerebral perfusion combined with cerebral vascular analysis, that is, cerebral perfusion data and multiphase vascular data may be obtained by signal scanning. However, arterial stage data cannot be automatically recognized in the current perfusion scanning, which can only reconstruct thin slice data of all the phase for vascular analysis, and cause a very large amount of data (e.g., a count of vascular vessel sequence images with a thickness of 0.5mm or 1mm may be up to 6000). Taking into account a reconstruction time, a transmission time, a time of loading into vascular vessel analysis and pretreatment, a time for vascular vessel evaluation may be long. In fact, a user may complete an assessment of vascular occlusion by only one phase of the arterial stage data, rather than reconstructing all the phases. According to the current traditional method, if only reconstructing one phase, the user needs to manually confirm which phase requires sequence splitting and manual archiving, which may cost a long time. Therefore, it is desirable to provide methods and systems for vascular analysis.

On the other hand, since the perfusion data is calculated based on data acquired at the plurality of consecutive time points, the data at each time point of the plurality of time points may remain consistent in structure, and the user can not have a large motion amplitude, even if the large motion amplitude happens after the motion calibration, the data may fail to match, resulting in calculation errors. The conventional perfusion acquisition needs to last at least 1 minute, since the time of acquisition is long, the body of the user may exist uncontrollable motion, and the large motion amplitude may appear at some time points, which may affect the accuracy of calculation. When the large motion amplitude occurs in the current perfusion scanning, generally, the user may identify and evaluate a motion situation independently, and manually judge whether the data of unqualified time points may be eliminated and recalculated. However, the user needs to be trained in advance, which may cause a high training cost, high requirements for the user, and a large human dependence, resulting in unsatisfactory data accuracy. Therefore, it is desirable to provide methods and systems for perfusion analysis that can automatically evaluate the motion amplitude and eliminate unqualified data.

Document US2021/153830 discloses a method for vascular analysis in which a peak phase is determined.

### SUMMARY

The invention is defined in independent claims 1, 14 and 15. Specific embodiments are defined in the dependent claims.

### BRIEF DESCRIPTION OF THE DRAWINGS

The present disclosure is further illustrated in terms of exemplary embodiments, and these exemplary embodiments are described in detail with reference to the drawings. These embodiments are not restrictive. In these embodiments, the same number indicates the same structure, wherein:
FIG. 1 is a schematic diagram illustrating an application scenario of a system for data analysis based on perfusion scanning according to some embodiments of the present disclosure;
FIG. 2 is a flowchart illustrating an exemplary process for vascular analysis according to some embodiments of the present disclosure;
FIG. 3 is a flowchart illustrating an exemplary process for determining a peak phase according to some embodiments of the present disclosure;
FIG. 4 is a flowchart illustrating an exemplary process for vascular analysis according to other embodiments of the present disclosure;
FIG. 5 is a flowchart illustrating an exemplary image reconstruction according to some embodiments of the present disclosure;
FIG. 6 is a flowchart illustrating an exemplary process for initial image reconstruction according to some embodiments of the present disclosure;
FIG. 7 is a flowchart illustrating an exemplary process for perfusion analysis according to some embodiments of the present disclosure;
FIG. 8 is a flowchart illustrating an exemplary determination process for removing perfusion scanning data according to some embodiments of the present disclosure;
FIG. 9 is a block diagram illustrating an exemplary system for data analysis based on perfusion scanning according to some embodiments of the present disclosure;
FIG. 10 is a structural diagram illustrating an exemplary device for data analysis based on perfusion scanning according to some embodiments of the present disclosure; and
FIG. 11 is a schematic diagram illustrating an exemplary time attenuation curve according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the accompanying drawing in the following description is merely some examples or embodiments of the present disclosure, for those skilled in the art, the present disclosure may further be applied in other similar situations according to the drawings without any creative effort. Unless obviously obtained from the context or the context illustrates otherwise, the same numeral in the drawings refers to the same structure or operation.

It should be understood that "system," "device," "unit" and/or "module" used in this specification is used for distinguishing different components, elements, parts or assemblies at different levels. However, if other words can achieve the same purpose, the above-mentioned words may be replaced by other expressions.

As used in the disclosure and the appended claims, the singular forms "a," "an," and "the" include plural referents unless the content clearly dictates otherwise. Generally speaking, the terms "comprise" and "include" only imply that the clearly identified steps and elements are included, and these steps and elements may not constitute an exclusive list, and the method or device may further include other steps or elements.

As shown in the present disclosure and claims, unless the context clearly indicates exceptions, the words "a," "an," "one," and/or "the" do not specifically refer to the singular, but may also include the plural. The terms "including" and "comprising" only suggest that the steps and elements that have been clearly identified are included, and these steps and elements do not constitute an exclusive list, and the method or device may also include other steps or elements.

The flowcharts used in the present disclosure illustrate operations that the system implements according to the embodiment of the present disclosure. It should be understood that a previous operation or a subsequent operation of the flowcharts may not be accurately implemented in order. Instead, a plurality of steps may be processed in reverse or simultaneously. Moreover, other operations may further be added to these procedures, or one or more steps may be removed from these procedures.

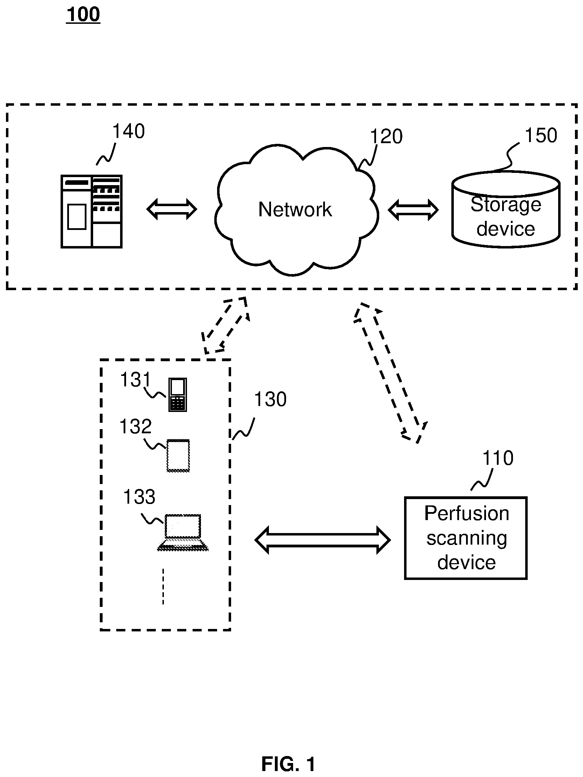

FIG. 1 is a schematic diagram illustrating an application scenario of a system for data analysis based on perfusion scanning according to some embodiments of the present disclosure. In some embodiments, a data analysis system 100 based on perfusion scanning may be applied to vascular analysis, the data analysis system 100 based on perfusion scanning may be referred to as a vascular analysis system. In some embodiments, the data analysis system 100 based on the perfusion scanning may be applied to the perfusion analysis, the data analysis system 100 based on the perfusion scanning may be referred to as a perfusion analysis system.

In some embodiments, the system 100 may include a perfusion scanning device 110, a network 120, one or more terminal device 130, a processing device 140, and a storage device 150. In some embodiments, one or more components in an application scenario of the system 100 may be connected directly or via the network 120.

The perfusion scanning device 110 may be configured to scan a target object using highenergy rays (e.g., an X-ray, a gamma ray, etc.) to collect perfusion scanning data related to the target object. In some embodiments, the perfusion scanning device 110 may include an ultrasound imaging (US) device, a computed tomography (CT) scanner, a digital radiography (DR) scanner (e.g., a mobile digital radiography), a digital subtraction angiography (DSA) scanner, a dynamic spatial reconstruction (DSR) scanner, an X-ray microscope scanner, a multi-modality scanner, etc., and/or any combination thereof. In some embodiments, the multi-modality scanner may include a computed tomography-positron emission tomography (CT-PET) scanner, a computed tomography-magnetic resonance imaging (CT-MRI) scanner, a single photon emission computed tomography-computed tomography (SPECT-CT) scanner. The target object may be biological or non-biological. For example, the target object may include a patient, a man-made object (e.g., a man-made phantom), etc. As another example, the target object may include a specific part, organ, and/or tissue of the patient.

The network 120 may include any suitable network that facilitates exchange of information and/or data for the system 100. In some embodiments, one or more components of system 100 (e.g., the perfusion scanning device 110, the terminal device 130, the processing device 140, and/or the storage device 150) may communicate information and/or via the network 120 with the one or more components of system 100. For example, the processing device 140 may obtain the perfusion scanning data from the perfusion scanning device 110 via the network 120. The network 120 may include a wired network and/or wireless network.

The terminal device 130 may include a mobile device 131, a tablet computer 132, a laptop computer 133, etc., or any combination thereof. In some embodiments, the mobile device 131 may include a smart home device, a wearable device, a mobile device, a virtual reality device, an augmented reality device, etc., or any combination thereof. In some embodiments, the terminal device 130 may be a part of the processing device 140.

The processing device 140 may process data and/or information obtained from the perfusion scanning device 110, the terminal device 130, and/or the storage device 150. For example, the processing device 140 may determine a peak phase in a plurality of phases based on perfusion scanning data of the plurality of phases; obtain a reconstruction result by performing, based on the perfusion scanning data of the plurality of phases, image reconstruction; and perform vascular analysis based on the reconstruction result. In some embodiments, the processing device 140 may be a single server or server group. The server group may be centralized or distributed. In some embodiments, the processing device 140 may be local or remote. For example, the processing device 140 may access information and/or data stored in the perfusion scanning device 110, the terminal device 130, and/or the storage device 150. As another example, the processing device 140 may access information and/or data by connecting with the perfusion scanning device 110, the terminal device 130, and/or the storage device 150. In some embodiments, the processing device 140 may be implemented on a cloud platform.

The storage device 150 may store data, instructions, and/or any other information. In some embodiments, the storage device 150 may store data obtained from the perfusion scanning device 110, the terminal device 130, and/or the processing device 140. For example, the storage device 150 may store the perfusion scanning data obtained from the perfusion scanning device 110. As another example, the storage device 150 may store data (e.g., the peak phase in the plurality of phases, the reconstruction result of the image reconstruction, etc.) generated by the processing device 140. In some embodiments, the storage device 150 may store the data and/or instructions of the exemplary methods performed by the processing device 140 as described in the present disclosure. In some embodiments, the storage device 150 may include a mass storage, a removable memory, a volatile read-write memory, a read-only memory (ROM), etc., or any combination thereof. The exemplary mass storage may include a magnetic disk, an optical disk, a solid-state disk, or the like. In some embodiments, the storage device may be implemented on a cloud platform. In some embodiments, the storage device 150 may communicate with the one or more components (e.g., the processing device 140 and/or the terminal device 130) of the system 100 via the network 120. In some embodiments, the storage device 150 may be a part of the processing device 140.

The description of the application scenario of the system 100 is intended to be illustrative, and not to limit the scope of the present disclosure. It should be noted that the above description is merely provided for the purposes of illustration, and not intended to limit the scope of the present disclosure. For persons having ordinary skills in the art, multiple variations and modifications may be made under the teachings of the present disclosure. After understanding the principle of the system, it is possible to make any combination of modules without departing from this principle, or form a subsystem to connect with other modules. In some embodiments, for example, each module may share a storage module, and each module may also have its own storage module. The features, structures, methods, and other features of the exemplary embodiments described herein may be combined in various ways to obtain additional and/or alternative exemplary embodiments. For example, the processing device 140 and the perfusion scanning device 110 may be integrated into a single device. However, those variations and modifications do not depart from the scope of the present disclosure.

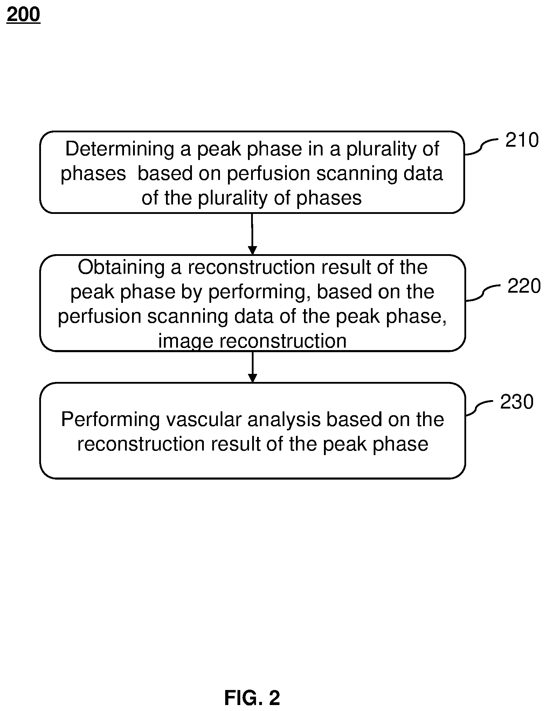

FIG. 2 is a flowchart illustrating an exemplary process 200 for vascular analysis according to some embodiments of the present disclosure.

In some embodiments, the vascular analysis process may be configured to perform vascular analysis based on perfusion scanning data. In some embodiments, an execution object performing the process 200 may include a perfusion scanning device and/or a controller. In some embodiments, the perfusion scanning device may be a medical imaging device, which may include at least one of a Computer Tomography (CT), a Nuclear Magnetic Resonance Imaging (MRI), an X-ray device, a Positron Emission Computed Tomography (PET), and an ultrasonic testing device. In some embodiments, the controller may be part of the system integrated into an electronic device, or an independent electronic device, and the controller may also be set in an online server. For example, the controller may be a personal computer, a laptop, a smartphone, a tablet, a portable wearable, or a system integrated into a medical imaging device (such as a central control device). The process 200 may be performed by the processing device 140, a data analysis system 900 based on the perfusion scanning, or a data analysis device 1000 based on the perfusion scanning.

In some embodiments, the process 200 may include the following operations.

In 210, a peak phase in a plurality of phases may be determined based on perfusion scanning data of a plurality of phases. In some embodiments, the operation 210 may be performed by a peak phase determination module 910.

The perfusion scanning data may refer to data obtained after performing perfusion scanning. For example, the perfusion scanning data may include original data obtained by the perfusion scanning, preprocessed data, etc. In some embodiments, the system 900 may obtain the perfusion scanning data of the plurality of time points by performing the perfusion scanning, and the perfusion scanning may include a scanning manner that continuously scans the plurality of phases (also referred to as time points) such as non-enhanced scanning and helical scanning. In some embodiments, the obtained perfusion scanning data may be perfusion scanning data of a plurality of consecutive phases, or perfusion scanning data of all the phases in the scanning time.

In some embodiments, before performing the perfusion scanning, some scanning preparation may be done. For example, the scanning preparation may include registering a patient who need the perfusion scanning and entering information of the patient. The information of the patient may include the name, gender, age, height, weight, of the patient, etc. In some embodiments, the scanning preparation may further include: setting a scanning interval, a scanning time, and a dose parameter of each phase of the perfusion scanning. For example, a scanning interval of the arterial stage may be set as 1.5-2 seconds, a scanning interval of an inflow and outflow stage may be set as 3-4 seconds, a transmission time of the arterial stage may be set as 15-30 seconds after the start of scanning, and a total scanning time may be set as not less than 60 seconds. The scanning interval, the transmission time, and the dose parameter of the arterial stage, and the scanning interval, the transmission time, and the dose parameter of the inflow and outflow stage may be preset in the execution object or set by the user, or further set by the user by performing modification within a reference data range given by the execution object.

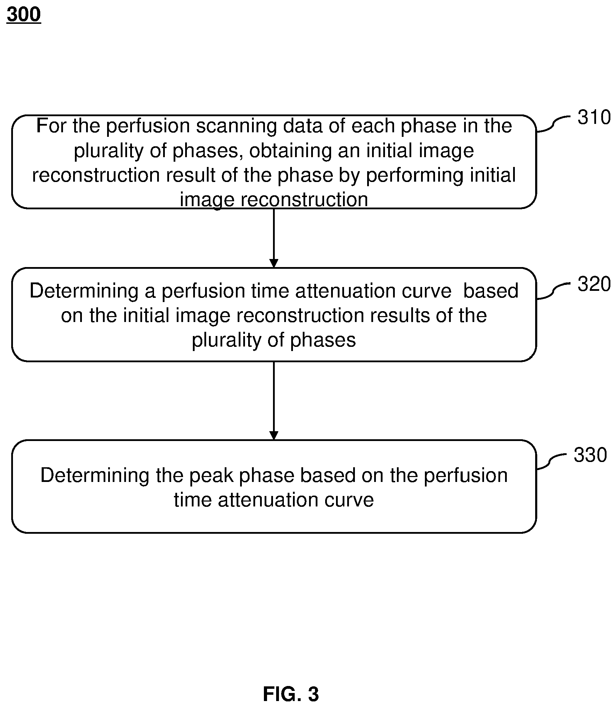

In some embodiments, the peak phase determination module 910 may obtain a reconstruction result by performing, based on the perfusion scanning data of the plurality of phases, image reconstruction. The peak phase determination module 910 may determine a perfusion time attenuation curve based on initial image reconstruction results of the plurality of phases. The peak phase determination module 910 may determine the peak phase based on a perfusion time attenuation curve. More descriptions about determining the peak phase may refer to FIG. 3 and the related descriptions. In some embodiments, performing the image reconstruction and the vascular analysis based on data of the peak phase may assist the user to perform vascular assessment (e.g., an assessment of vascular occlusion) better.

In 220, a reconstruction result of the peak phase may be obtained by performing, based on the perfusion scanning data of the peak phase, image reconstruction. In some embodiments, the operation 220 may be performed by an image reconstruction module 920.

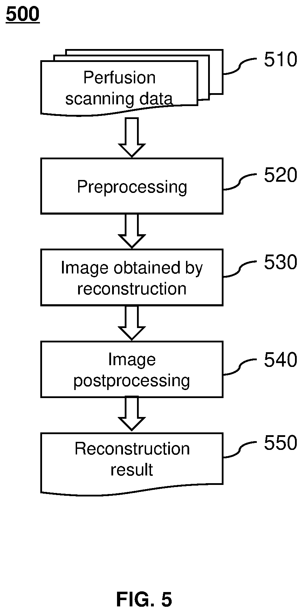

In some embodiments, the image reconstruction module 920 may preprocess the perfusion scanning data of the peak phase; then the image reconstruction module 920 may obtain a perfusion scanning image of the peak phase by reconstructing the preprocessed compressed perfusion scanning data; and the image reconstruction module 920 may obtain a reconstruction result of the peak phase by postprocessing the perfusion scanning image of the peak phase. More descriptions about the image reconstruction may refer to FIG.5 and the related descriptions.

In 230, vascular analysis may be performed based on the reconstruction result of the peak phase. In some embodiments, the operation 230 may be performed by a vascular analysis module 930.

In some embodiments, the vascular analysis module 930 may obtain a vascular analysis result by performing the vascular analysis automatically based on the reconstruction result of the peak phase. In some embodiments, the vascular analysis module 930 may perform vascular analysis preprocessing on the reconstruction result (e.g., an image after reconstruction). The vascular analysis preprocessing may include removing image information such as a bed plate and a bone in the image. In some embodiments, the vascular analysis preprocessing may be performed manually by the user and/or automatically by software. For example, the vascular analysis preprocessing may be performed by a preprocessing model (e.g., a machine learning model). In some embodiments, the vascular analysis module 930 may perform vascular extraction on the preprocessed image. During the vascular extraction, the vascular analysis module 930 may extract vascular information (e.g., an image, location, and size of the vascular) in the image by identifying a centerline and contour of the vascular vessel automatically based on a trained image identification model (e.g., a machine learning model). In some embodiments, after completing the vascular extraction, the user may confirm and/or modify the vascular extraction by manually editing the centerline of the vascular. In some embodiments, after completing the vascular extraction (or after the user confirms and/or modifies the vascular extraction), the vascular analysis module 930 may complete the vascular analysis operation and obtain a vascular analysis result by automatically performing quantitative analysis of lumen stenosis degree based on the extracted vascular information. In some embodiments, the vascular analysis result may include a quantitative analysis result of lumen stenosis degree.

In some embodiments, the vascular analysis result may be used by a user (e.g., a doctor) to assess the vascular occlusion of the patient. In some embodiments, the vascular analysis module 930 may upload the vascular vessel analysis result to a Picture Archiving and Communication Systems (PACS).

In some embodiments, the vascular analysis module 930 may determine whether a motion amplitude of the peak phase is greater than a preset amplitude based on the reconstruction result of the peak phase; and in response to determining that the motion amplitude of the peak phase is less than or equal to the preset amplitude, the vascular analysis module 930 may perform the vascular analysis based on the reconstruction result of the peak phase.

The motion amplitude of the phase may refer to a motion amplitude of a scanning site (e.g., a head, a brain, etc.) in the phase, and the motion amplitude of the scanning site in the phase may be reflected by the perfusion scanning data of the phase. When the motion amplitude of the scanning site in the phase is large, the perfusion scanning data of the phase may be abnormal (e.g., the image appears artifacts), or an object in the perfusion scanning image of the phase may deviate from an image of an adjacent phase. In some embodiments, the preset amplitude may refer to a maximum allowable motion amplitude of the scanning site. In response to determining that the motion amplitude of the peak phase is less than or equal to the preset amplitude, the vascular analysis may be performed based on the reconstruction result.

In some embodiments, in response to determining that the motion amplitude of the peak phase is greater than the preset amplitude, the vascular analysis module 930 may prompt the user for relevant information of the motion amplitude of the peak phase by generating prompting information.

In some embodiments, the prompting information may reflect the relevant information of the motion amplitude of the peak phase. The prompting information may be one or more forms of information such as text, voice, and image. In some embodiments, the prompting information may be displayed to the user in various forms through the one or more terminal devices130 or through the perfusion scanning device 110. By prompting the user that the motion amplitude of the peak phase is greater than the preset amplitude, the user may timely understand the motion amplitude of the peak phase, so as to assist the user to make correct analysis and judgment.

In some embodiments, in response to determining that the motion amplitude of the peak phase is greater than the preset amplitude, the vascular analysis module 930 may obtain a reconstruction result of an adjacent phase of the peak phase by performing, based on the perfusion scanning data of the adjacent phase of the peak phase, image reconstruction; and the vascular analysis may be performed based on the reconstruction result of the peak phase and the reconstruction result of the adjacent phase of the peak phase.

The adjacent phase may be one or more phases adjacent to the peak phase. A range of the adjacent phase may be determined by presetting. For example, the adjacent phase may be one or more phases within 2 seconds before and/or after the peak phase. In some embodiments, the vascular analysis module 930 may further determine whether the motion amplitude of the peak phase is greater than the preset amplitude and obtain the reconstruction result of the adjacent phase by performing the image reconstruction on the perfusion scanning data of the adjacent phase that are less than the preset amplitude.

In some embodiments, the vascular analysis module 930 may comprehensively perform the vascular analysis based on the reconstruction result of the peak phase and the reconstruction result of the adjacent phase. By comprehensively performing analysis by introducing adjacent phases of the peak phase, when the motion amplitude of the peak phase is greater than the preset amplitude, compensation analysis may be performed by introducing the perfusion scanning data of the adjacent phases, which may ensure the accuracy of the vascular analysis result.

In the vascular analysis method provided in the embodiments of the present disclosure, the peak phase in the plurality of phases may be determined automatically, and the reconstruction result of the peak phase may be obtained by performing, based on the perfusion scanning data of the peak phase, the image reconstruction, and the vascular analysis may be performed based on the reconstruction result. The vascular analysis method can reduce unnecessary data processing and analysis, reduce the time-consuming of vascular analysis evaluation, and improve the evaluation efficiency, which can shorten the time of core index cerebral stroke (DNT) of stroke center effectively and be beneficial to clinical application.

FIG. 3 is a flowchart illustrating an exemplary process for determining a peak phase according to some embodiments of the present disclosure. As shown in FIG. 3, a peak phase determination process 300 may include the following operations.

In 310, for the perfusion scanning data of each phase in the plurality of phases, an initial image reconstruction result of each phase may be obtained by performing initial image reconstruction on the perfusion scanning data of each phase in the plurality of phases, respectively. In some embodiments, the operation 310 may be performed by an image reconstruction module 920.

In some embodiments, a reconstructed slice thickness of the initial image reconstruction is greater than a reconstructed slice thickness of the image reconstruction in the operation 220. In some embodiments, the reconstructed slice thickness of the image reconstruction in the operation 220 may be 1mm; and the reconstructed slice thickness of the initial image reconstruction may be 5mm. By using a relatively large slice thickness for the initial image reconstruction, it is beneficial to obtain the initial image reconstruction result of each of the plurality of phases quickly. In some embodiments, the reconstructed slice thickness of the initial image reconstruction may be greater than or equal to 3mm; and the reconstructed slice thickness of the image reconstruction in the operation 220 may be less than 3 mm. For example, the reconstructed slice thickness of the initial image reconstruction may be 5mm. The reconstructed slice thickness of the image reconstruction in the operation 220 may be 2mm, 1.5mm, 1mm, etc.

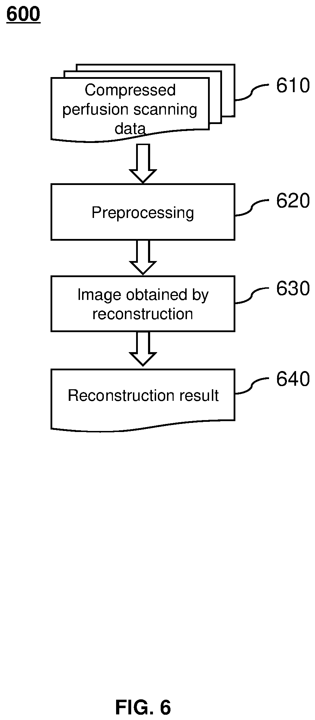

In some embodiments, a count of reconstruction operations of the initial image reconstruction may be less than a count of reconstruction operations of the image reconstruction. In some embodiments, the image reconstruction module 920 may preprocess the perfusion scanning data of each phase; the image reconstruction module 920 may obtain a perfusion scanning image of each phase and the reconstruction result of the initial image reconstruction by reconstructing the preprocessed perfusion scanning data of each phase. More descriptions about the initial image reconstruction may refer to FIG. 6 and the related descriptions. By using relatively few operations for the initial image reconstruction (if not perform the postprocessing), it is beneficial to obtain the initial image reconstruction result of each of the plurality of phases phase quickly. In some embodiments, the initial image reconstruction may be performed based on compressed perfusion scanning data of each phase, thereby further improving the efficiency of the initial image reconstruction.

In some embodiments, the reconstructed slice thickness of the initial image reconstruction may be greater than the reconstructed slice thickness of the image reconstruction in the operation 220, and the reconstruction operations of the initial image reconstruction may be less than the reconstruction operations of the image reconstruction in the operation 220.

In 320, a perfusion time attenuation curve may be determined based on the initial image reconstruction results of the plurality of phases. In some embodiments, the operation 320 may be performed by the peak phase determination module 910.

In some embodiments, the peak phase determination module 910 may determine the perfusion time attenuation curve by performing the perfusion analysis on the initial image reconstruction results of the plurality of phases.

In some embodiments, the peak phase determination module 910 may determine the perfusion time attenuation curve based on image data of an arterial region in the initial image reconstruction results. In some embodiments, the peak phase determination module 910 may identify the arterial region based on the initial image reconstruction results of the plurality of phases. The peak phase determination module 910 may further determine a perfusion time attenuation curve of the arterial region based on the image data of the arterial region in the initial image reconstruction results of the plurality of phases. The arterial region may be a region including an arterial location. In some embodiments, the arterial region may be the smallest region including an arterial location. In some embodiments, the peak phase determination module 910 may identify the arterial region of the initial image reconstruction result using a trained arterial region identification model (e.g., a machine learning model). In some embodiments, the peak phase determination module 910 may identify the arterial region for the initial image reconstruction result of a phase (e.g., a first phase), and determine a position corresponding to the arterial region of the phase (e.g., the first phase) in other phases as the arterial region of the other phases. Determining the perfusion time attenuation curve based on the image data of the arterial region in the initial image reconstruction results can effectively improve the efficiency of determining the perfusion time attenuation curve.

In 330, the peak phase may be determined based on the perfusion time attenuation curve. In some embodiments, the operation 330 may be performed by the peak phase determination module 910.

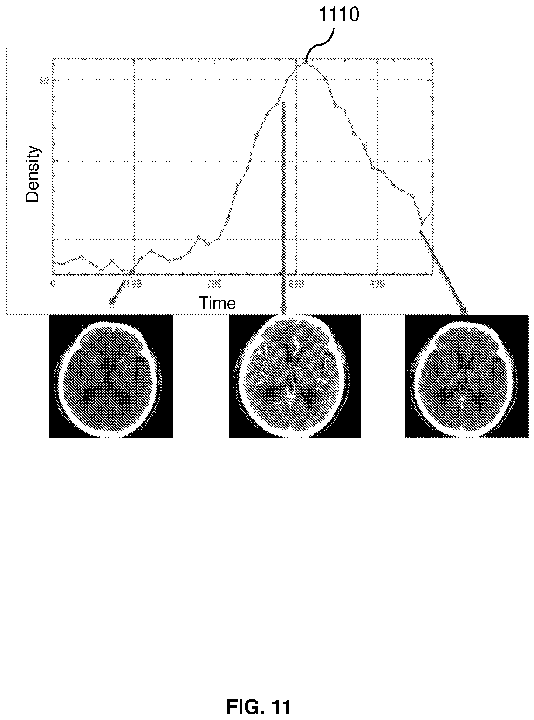

In some embodiments, the peak phase determination 910 may determine a phase corresponding to a peak time point in the perfusion time attenuation curve as the peak phase. FIG. 11 is a schematic diagram illustrating an exemplary time attenuation curve according to some embodiments of the present disclosure. The perfusion time attenuation curve may reflect a relationship between the density of contrast agent flowing through a perfusion scanning site and a perfusion scanning time. Taking the perfusion time attenuation curve as shown in FIG. 22 as an example, a phase corresponding to a peak time point 1110 in the perfusion time attenuation curve may be determined as the peak phase.

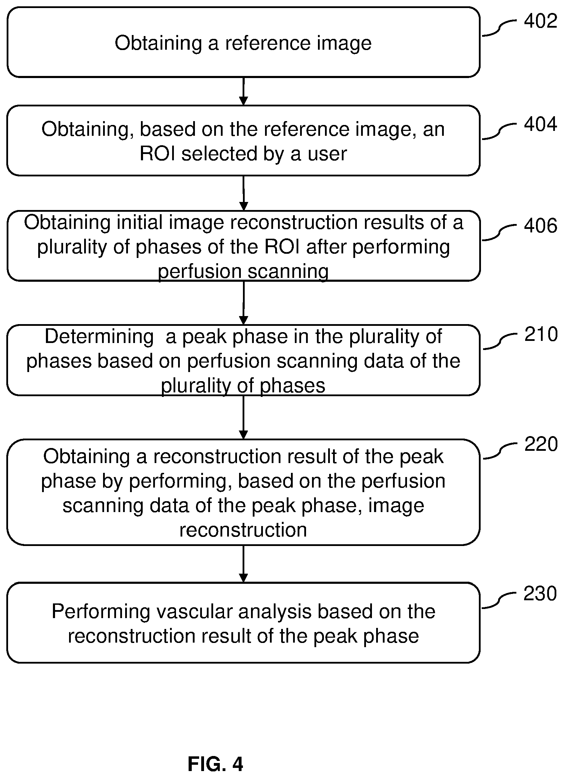

FIG. 4 is a flowchart illustrating an exemplary process for vascular analysis according to other embodiments of the present disclosure.

In some embodiments, the perfusion scanning data of the plurality of phases in the operation 210 may be perfusion scanning data of a region of interest (ROI). In some embodiments, as shown in FIG. 4, before performing the operation 210, the system 900 (e.g., the peak phase determination module 910) may perform operations 402-406, to obtain the perfusion scanning data of the plurality of phases of the region of interest.

In 402, a reference image may be obtained. The system 900 may obtain the reference image by controlling a perfusion scanning device (e.g., a CT device) to scan a scanning region.

In 404, a region of interest selected by a user may be obtained.

In some embodiments, the system 900 may transmit the reference image to a terminal device of the user (e.g., a medical staff), and the user may determine the region of interest in the reference image by the terminal device. For example, the user may outline the region of interest to be processed in the reference image in the form of box, circle, ellipse, irregular polygon, etc. In some embodiments, the user may select the arterial region as the region of interest. The system 900 may obtain the region of interest selected by the user.

In 406, after perfusion scanning is performed, the perfusion scanning data of the plurality of phases of the region of interest may be obtained. In some embodiments, the system 900 may control the perfusion scanning device to scan the region of interest only, and further obtain the perfusion scanning data of the plurality of phases of the region of interest. In some embodiments, the system 900 may perform the perfusion scanning on a relatively wide scanning range, and after performing the perfusion scanning, the perfusion scanning data of the plurality of phases of the region of interest may be obtained.

As shown in the embodiments of FIG. 4, in the operation 210, the peak phase determination module 910 may determine the peak phase in the plurality of phases based on the perfusion scanning data of the plurality of phases of the region of interest. In the operation 220, the image reconstruction module 920 may obtain a reconstruction result by performing the image construction based on the perfusion scanning data of the peak phase of the region of interest. In the operation 230, the vascular analysis module 930 may perform vascular analysis based on the reconstruction result. By predetermining the region of interest, and processing and analyzing the perfusion scanning data of the region of interest, the efficiency of vascular analysis can be effectively improved.

FIG. 5 is a flowchart illustrating an exemplary process for image reconstruction according to some embodiments of the present disclosure. In some embodiments, an image reconstruction process 500 may be performed by the image reconstruction module 920. As shown in FIG. 5, the process 500 may include the following operations.

In 510, perfusion scanning data may be obtained. In some embodiments, the perfusion scanning data may be the perfusion scanning data of the peak phase. In some embodiments, the perfusion scanning data may be the perfusion scanning data of the peak phase of the region of interest.

In 520, the perfusion scanning data may be preprocessed. In some embodiments, the preprocessing of the perfusion scanning data may include but is not limited to data cleaning, data integration, data transformation, or any combination thereof.

In 530, a perfusion scanning image may be obtained by reconstructing the preprocessed perfusion scanning data. In some embodiments, the perfusion scanning image of the peak phase may be obtained by reconstructing the preprocessed perfusion scanning data. In some embodiments, the perfusion scanning image of the peak phase may be obtained by performing reconstruction with a first slice thickness (e.g., 1.5mm, 1mm, 0.5mm, etc.) on the preprocessed perfusion scanning data. In some embodiments, the image reconstruction may include filtered back projection (FBP) image reconstruction.

In 540, the image obtained by reconstruction may be postprocessed. In some embodiments, the perfusion scanning image of the peak phase obtained by the reconstruction may be postprocessed. In some embodiments, the postprocessing may include but is not limited to an enhancement processing, an interpolation processing, a morphological processing, a noise removal, etc., or any combination thereof. In some embodiments, the postprocessing may include the enhancement processing, wherein the enhancement processing may highlight some structures or regions in an original image. The enhancement processing may include a square image equalization algorithm, a wavelet transform image enhancement algorithm, a partial differential equation image enhancement algorithm, a Hessian matrix enhancement algorithm, etc., or any combination thereof. In some embodiments, the postprocessing may include an interpolation processing, wherein the interpolation processing may make a voxel size uniform in the original image. In some embodiments, the postprocessing may include a morphological processing, wherein the morphological processing may use elements with a morphological structure to process shapes in the original image to achieve the purpose of analyzing and identifying the target. The morphological processing manner may include but is not limited to dilation, erosion, opening operation, closing operation, etc., or any combination thereof. In some embodiments, the postprocessing may include noise removal, wherein the noise removal may remove interference due to machine noise, target movement, etc., in the original image. The noise removal may include but is not limited to median filter, mean filter, etc., or any combination thereof. In some embodiments, the postprocessing may include other reasonable processing operations (e.g., stripping, calibration, etc.), which may not be limited in the present disclosure.

In 550, the postprocessed reconstruction result may be obtained. In some embodiments, the postprocessed reconstruction result may include a reconstruction result of the peak phase.

FIG. 6 is a flowchart illustrating an exemplary process for initial image reconstruction according to some embodiments of the present disclosure. In some embodiments, an initial image reconstruction process 600 may be performed by the image reconstruction module 920. As shown in FIG. 6, the process 600 may include the following operations.

In 610, compressed perfusion scanning data may be obtained. In some embodiments, the compressed perfusion scanning data may include compressed perfusion scanning data of each phase (or the perfusion scanning data of each phase of the region of interest). The compressed perfusion scanning data may be data obtained by compressing the perfusion scanning data using a compression algorithm. The exemplary compression algorithm may include lossy compression, lossless compression, discrete cosine change, quantization, neural networks, etc. Compressing raw perfusion scanning data can effectively reduce the amount of data, improve the efficiency of data processing, reduce the burden on system resources for data transmission and storage, and shorten a time lag between data acquisition and image display. In some embodiments, uncompressed perfusion scanning data may also be obtained.

In 620, the perfusion scanning data (e.g., the compressed perfusion scanning data) may be preprocessed. In some embodiments, the preprocessing of the perfusion scanning data may include but is not limited to data cleaning, data integration, data transformation, etc., or any combination thereof.

In 630, a perfusion scanning image may be obtained by reconstructing the preprocessed compressed perfusion scanning data. In some embodiments, the perfusion scanning image of each phase may be obtained by reconstructing the preprocessed perfusion scanning data of each phase. In some embodiments, the perfusion scanning image of each phase may be obtained by performing the reconstruction with a second slice thickness (e.g., 4mm, 5mm, 6mm, etc.) on the preprocessed perfusion scanning data of each phase. In some embodiments, the second slice thickness may be greater than the first slice thickness. In some embodiments, the image reconstruction may include filtered Back Projection (FBP) image reconstruction.

In 640, a reconstruction result of the initial image reconstruction may be obtained. In some embodiments, the initial image reconstruction process 600 may include but is not limited to a postprocessing operation, which may improve the efficiency of the initial image reconstruction.

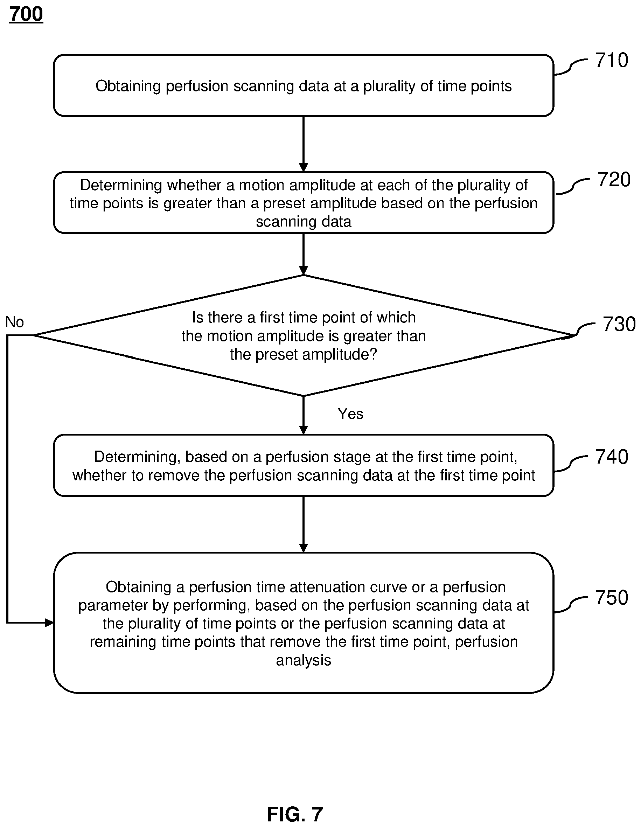

FIG. 7 is a flowchart illustrating an exemplary process 700 for perfusion analysis according to some embodiments of the present disclosure.

An execution object of the process 700 may include a perfusion scanning device and/or a controller. In some embodiments, the perfusion scanning device may be a medical imaging device, which may include at least one of a Computer Tomography (CT), a Nuclear Magnetic Resonance Imaging (MRI), an X-ray device, a Positron Emission Computed Tomography (PET), and an ultrasonic testing device. In some embodiments, the controller may be part of the system integrated into an electronic device, or an independent electronic device, and the controller may also be set in an online server. For example, the controller may be a personal computer, a laptop, a smartphone, a tablet, a portable wearable, or systems integrated into a medical imaging device (such as a central control device). In some embodiments, a perfusion analysis process 700 may be performed by the processing device 140, the system 900, or the device 1000.

In some embodiments, the process 700 may include the following operations.

In 710, perfusion scanning data at a plurality of time points may be obtained. In some embodiments, the operation 710 may be performed by an obtaining module 940.

The perfusion scanning data may refer to data obtained after perfusion scanning is performed. For example, the perfusion scanning data may include original data obtained by the perfusion scanning, the perfusion scanning image obtained by reconstruction, etc. In some embodiments, the perfusion scanning data at the plurality of time points may be obtained by performing the perfusion scanning, and the perfusion scanning may include a scanning manner that continuously scans the plurality of time points such as non-enhanced scanning and helical scanning. In some embodiments, the obtained perfusion scanning data may be the perfusion scanning data at the plurality of time points, or the perfusion scanning data at all the time points in the scanning time. In some embodiments, in the present disclosure, "time point" and "phase" may be used interchangeably.

In some embodiments, before the perfusion scanning is performed, one or more scanning preparations may be performed. For example, the scanning preparation may include registering a patient who needs the perfusion scanning and entering information of the patient. The information of the patient may include the name, gender, age, height, weight, of the patient, etc. In some embodiments, the scanning preparation may further include: performing a CT reference image scanning. In some embodiments, a user (e.g., an operator of a perfusion scanning project) may plan localized slices and large-scale slices of perfusion scanning (e.g., setting needed scanning regions). In some embodiments, the scanning preparation may further include: setting a scanning interval, a scanning time, and a dose parameter of each phase of the perfusion scanning. For example, a scanning interval of the arterial stage may be set as 1.5-2 seconds, a scanning interval of the inflow and outflow stage may be set as 3-4 seconds, a transmission time of the arterial stage may be set as 15-30 seconds after the start of scanning, and a total scanning time may be set as not less than 60 seconds. The scanning interval, the transmission time, and the dose parameter of the arterial stage, and the scanning interval, the transmission time, and the dose parameter of the inflow and outflow stage may be preset in the execution object or set by the user, or further set by the user by performing modification within a reference data range given by the execution object.

In some embodiments, the perfusion scanning data at the plurality of time points may be the perfusion scanning data of the region of interest. The region of interest may be a region selected by the user.

In some embodiments, the perfusion analysis module 970 may obtain the reference image; the region of interest selected by the user based on the reference image; and after the perfusion scanning is performed, the perfusion scanning data at the plurality of time points of the region of interest may be obtained. In some embodiments, the system 900 may obtain the reference image by controlling the perfusion scanning device (e.g., a CT device) to scan the scanning region.

In some embodiments, the system 900 may transmit the reference image to the terminal device of the user (e.g., a medical staff), and the user may determine the region of interest in the reference image by the terminal device. For example, the user may outline the region of interest to be processed in the reference image in the form of box, circle, ellipse, irregular polygon, etc. In some embodiments, the user may select the arterial region as the region of interest. The system 900 may obtain the region of interest selected by the user. In some embodiments, the perfusion scanning data may be the perfusion scanning data of all the scanning regions.

In some embodiments, the system 900 may obtain the perfusion scanning data at the plurality of time points of the region of interest by controlling the perfusion scanning device to scan the region of interest. In some embodiments, the system 900 may perform the perfusion scanning on a relatively wide range scanning range, after the perfusion scanning is performed, the perfusion scanning data at the plurality of time points of the region of interest may be obtained. By obtaining the perfusion scanning data of the region of interest, the interference of other unnecessary information on the analysis results may be avoided, and the efficiency and accuracy of perfusion analysis may be improved.

In 720, a determination of whether a motion amplitude at each of the plurality of time points is greater than a preset amplitude may be determined based on the perfusion scanning data. In some embodiments, the operation 720 may be performed by a motion amplitude abnormality determination module 950.

The motion amplitude at a time point may refer to a motion amplitude of the scanning site at the time point, the motion amplitude of the scanning site at the time point may reflect by the perfusion scanning data at the time point. When the motion amplitude at the time point is too large, the perfusion scanning data at the time point may be abnormal (e.g., the image appears artifacts), or the object in the perfusion scanning image at the time point may deviate from an image of the adjacent phase. In some embodiments, the preset amplitude may refer to a maximum allowable motion amplitude of the scanning site.

In some embodiments, the motion amplitude abnormality determination module 950 may obtain a variation between the perfusion scanning image at each time point and the perfusion scanning image at an adjacent time point, and determine, based on the variation between the perfusion scanning image at each time point and the perfusion scanning image at the adjacent time point, whether the motion amplitude of each of plurality of time points is greater than the preset amplitude. The variation may represent a feature quantity of a difference between the perfusion scanning image at the time point (e.g., the scanning object in the image) and the perfusion scanning images at the adjacent time point. For example, the variation may include a rotation variable and/or a translation variable, etc. In some embodiments, the variation may represent a rotation variable and/or a translation variable. Whether the motion amplitude at the time point is greater than the preset amplitude may be evaluated based on the rotation variable and the translation variable of the perfusion scanning image at the time point and the perfusion scanning image at the adjacent time point. In some embodiments, the motion amplitude abnormality determination module 950 may select an image at a time point as a reference image, and the rotation variable and translation variable between the images may be obtained by registering an image at other time point (e.g., an adjacent time point) that is designated as a floating image.

In some embodiments, the motion amplitude abnormality determination module 950 may establish a preset threshold of the variation, the preset threshold may refer to a maximum value that can be reached by the variation within a preset amplitude. In some embodiments, when the variation between the time point and the adjacent time point is greater than the preset threshold, it may be determined that the motion amplitude at the time point is greater than the preset threshold. When at least one of variations between the time point and adjacent time points is less than or equal to the preset threshold, it may be determined that the motion amplitude at the time point is not greater than the preset threshold. An adjacent time point prior to the time point may refer to a time point adjacent to the time point and before the time point, and an adjacent time point after the time point may refer to a time point adjacent to the time point and after the time point. In some embodiments, the first time point and the last time point of the plurality time points may not be judged (e.g., the first time point and the last time point of the plurality time points may be regarded as not being adjacent).

In some embodiments, the preset threshold may include a maximum value that the rotation variable and the translation variable can reach respectively within the preset amplitude. In some embodiments, when the rotation variables and the translation variables of the perfusion scanning images at the time point and the perfusion scanning images at the adjacent time points prior to or after the time point are greater than the preset threshold, it may be determined that the motion amplitude at the time point is greater than the preset amplitude. When at least one of the rotation variables and the translation variables of the perfusion scanning images at the time point and the perfusion scanning images at the adjacent time points prior to or after the time point is less than or equal to the preset threshold, it may be determined that the motion amplitude at the time point is not greater than the preset amplitude. In some embodiments, when at least one of the rotation variables and the translation variables of the perfusion scanning images at the time point and the perfusion scanning images at the time points prior to or after the time point is greater than the preset threshold, it may be determined that the motion amplitude at the time point is greater than the preset amplitude. when the rotation variables and the translation variables of the perfusion scanning images at the time point and the perfusion scanning images at the adjacent time points prior to or after the time point are less than or equal to the preset threshold, it may be determined that the motion amplitude at the time point is not less than the preset amplitude. In some embodiments, the preset threshold may be preset by a user (e.g., those skilled in the art). In some embodiments, the preset threshold may be automatically determined according to historical data.

In some embodiments, the variable may be represented as mutual information between two images, and the mutual information may represent a similarity between two images. Through the mutual information of the perfusion scanning image at the time point and the perfusion scanning image at the adjacent time point, the motion amplitude at the time point may be evaluated. In some embodiments, the preset threshold may refer to a lowest value (e.g., a lowest similarity) that the mutual information can achieve within a preset amplitude. When the mutual information of the perfusion scanning image at the time point and the perfusion scanning images at the time point prior to or after the time point is less than the preset threshold, it may be determined that the motion amplitude at the time point is greater than the preset amplitude. When at least one of the mutual information of the perfusion scanning image at the time point and the perfusion scanning images at the time point prior to or after the time point is less than or equal to the preset threshold, it may be determined that the motion amplitude at the time point is not greater than the preset amplitude.

In some embodiments, the motion amplitude abnormality determination module 950 may determine whether the motion amplitude at each of the plurality of time points is greater than the preset amplitude using the trained motion amplitude abnormality determination module based on the perfusion scanning image at each of the plurality of time points. In some embodiments, the motion amplitude abnormality determination module may be a machine learning model. In some embodiments, the trained motion amplitude abnormality determination module may be obtained by building a training set to train the machine learning model. The training set may include scanning image data that the motion amplitude is greater than the preset amplitude and scanning image data that the motion amplitude is not greater than the preset amplitude. The motion amplitude abnormality determination model may be obtained by performing a secondary classification training on the training set. The motion amplitude abnormality determination model may be used to determine whether the motion amplitude of the perfusion scanning image at each of the plurality of time points is greater than the preset amplitude.

In 730, a determination of whether a motion amplitude at a first time point of the plurality of time points is greater than the preset amplitude may be determined. In some embodiments, the operation 730 may be performed by the motion amplitude abnormality determination module 950.

In some embodiments, the motion amplitude abnormality determination module 950 may determine the time point that the motion amplitude is greater than the preset amplitude as the first time point. For example, when the variable of the perfusion scanning image at the time point and the perfusion scanning image at the adjacent time point prior to or after the time point is greater than the preset threshold, it may be determined the time point as a first time point that the motion amplitude is greater than the preset threshold. When the motion amplitude at the first time point is greater than the preset amplitude, operation 740 may be performed based on the system 900.

In some embodiments, when the motion amplitude of any time point is not less than the preset amplitude, the motion amplitude abnormality determination module 950 may determine that there is no first time point. When there is no first time point, operation 750 may be performed based on the system 900.

In 740, a determination of whether to remove the perfusion scanning data at the first time point may be determined based on a perfusion stage at the first time point. In some embodiments, the operation 740 may be performed by a data removing module 960.

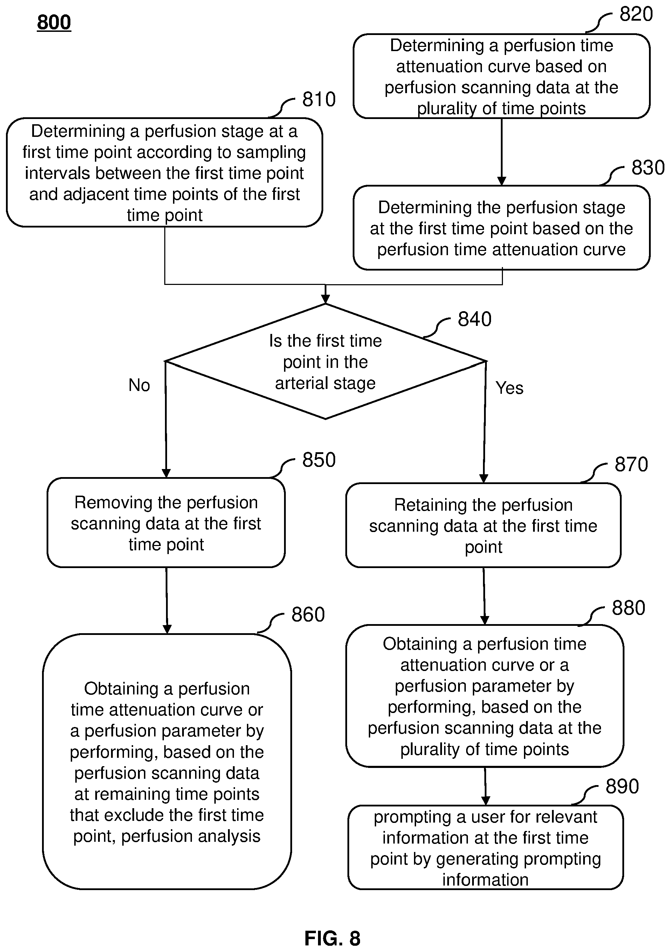

In some embodiments, the perfusion stage may be divided according to the flow of injected contrast medium in the vascular. In some embodiments, the perfusion stage may include a flow direction of the contrast agent, inflow and outflow stage of the outflow scanning region (e.g., body parts such as a head or heart), and an arterial stage that a large dose of contrast agent is concentrated on the arterial vascular in the scanning region. In some embodiments, when the first time point is in the arterial stage, the data removing module 320 may not remove the perfusion scanning data at the first time point (e.g., retain the perfusion scanning data at the first time point). In some embodiments, when the first time point is in the inflow and outflow stage, the data removing module 960 may remove the perfusion scanning data at the first time point. More descriptions about the data remove may refer to FIG. 8 and the related descriptions.

In 750, a perfusion time attenuation curve and/or perfusion parameter may be obtained by performing, based on the perfusion scanning data at the plurality of time points or the perfusion scanning data at remaining time points that remove the first time point, perfusion analysis. In some embodiments, the operation 750 may be performed by a perfusion analysis module 970.

In some embodiments, the perfusion parameter may include but is not limited to cerebral blood volume (CBF), local cerebral blood volume (CBV), mean transit time (MTT), time to peak (TTP), etc., or any combination thereof. In some embodiments, when there is no first time point that the motion amplitude is greater than the preset amplitude or there is the first time point that does not need to be removed, the perfusion analysis module 970 may obtain the perfusion time attenuation curve and/or perfusion parameter by performing the perfusion analysis based on the perfusion scanning data at the plurality of time points. In some embodiments, when there is the first time point of which the motion amplitude is greater than the preset amplitude and that can be removed, the perfusion analysis module 970 may obtain the perfusion time attenuation curve and/or perfusion parameter by performing, based on the perfusion scanning data at remaining time points that remove the first time point, the perfusion analysis. In some embodiments, when the perfusion stage at the first time point is determined based on the time attenuation curve, an initial time attenuation curve may be generated based on the perfusion scanning data at the plurality of time points, and the perfusion stage at the first time point may be determined based on the initial time attenuation curve. When not removing the perfusion scanning data at the first time point, the perfusion analysis module 970 may determine the initial time attenuation curve as a final initial time attenuation curve. When removing the first time point, the perfusion analysis module 970 may regenerate the final initial time attenuation curve according to the perfusion scanning data at the remaining time point that exclude the first time point.

In the perfusion analysis method provided by embodiments of the present disclosure, the time point that the motion amplitude is too large may be determined by evaluating the motion amplitude at each of the plurality of time points, and whether to remove the perfusion scanning data at the first time point may be determined according to the perfusion stage at the time point. The present disclosure may determine the accuracy of the perfusion scanning data automatically under an excessive motion amplitude of the patient, analyze and process unqualified perfusion scanning data, and give the user relatively more accurate results, which can reduce the dependence on human judgment and reduce the training cost for the user.

FIG. 8 is a flowchart illustrating an exemplary determination process for removing data according to some embodiments of the present disclosure. In some embodiments, when the motion amplitude at the first time point is greater than the preset amplitude, the system 900 may perform a determination process 800 for removing perfusion scanning data, and further determine whether to remove the perfusion scanning data at the first time point according to the perfusion stage at the first time point. In some embodiments, operations in the process 800 may be performed by the data removing module 960 and/or the perfusion analysis module 970.

In 810, the perfusion stage at the first time point may be determined according to sampling intervals between the first time point and adjacent time points of the first time point. In some embodiments, the operation 810 may be performed by the data removing module 960.

In some embodiments, when the sampling intervals between the first time point and adjacent time points prior to or after the first time point are greater than a preset interval, the data removing module 960 may determine that the first time point is in the inflow and outflow stage. In some embodiments, when at least one of the sampling intervals between the first time point and adjacent time points prior to or after the first time point is less than or equal to the preset interval, the data removing module 960 may determine that the first time point is in the arterial stage.

In some embodiments, the present interval may be set according to the scanning interval set before the scanning. Since a scanning interval in the arterial stage is less than a scanning interval in the inflow and outflow stage, the preset interval may be set as the scanning interval in the arterial stage, a scanning interval slightly less than the inflow and outflow stage, or a value between the scanning interval in the arterial stage and the scanning interval slightly less than the inflow and outflow stage. For example, the scanning interval in the arterial stage may be 1.5 seconds, the scanning interval slightly less than the inflow and outflow stage may be 3 seconds, the preset interval may be 1.5 seconds, 2.9 seconds, 2.5 seconds, etc.

In some alternative embodiments, the preset interval may be set as the scanning interval in the inflow and outflow stage, when the sampling intervals between the first time point and adjacent time points prior to or after the first time point are equal to the preset interval, it may be determined that the first time point is in the inflow and outflow stage; when at least one of the sampling intervals between the first time point and adjacent time points prior to or after the first time point is less than the preset interval, it may be determined that the first time point is in the arterial stage.

By determining the perfusion stage at the first time point based on the sampling intervals between the first time point and adjacent time points of the first time point, the perfusion stage at the first time point may be determined quickly, and whether to remove the perfusion scanning data at the time point may be determined, which can improve the speed and accuracy of the perfusion analysis.

In some embodiments, the data removing module 960 may determine the perfusion stage at the first time point by performing the operation 810. In some embodiments, the data removing module 960 may determine the perfusion stage at the first time point by performing the operations 820 and 830.

In 820, the time attenuation curve may be determined based on the perfusion scanning data at the plurality of time points.

The time attenuation curve may reflect a relationship between the density of the contrast agent flowing through a perfusion scanning site and a perfusion scanning time. FIG. 11 is a schematic diagram illustrating an exemplary time attenuation curve according to some embodiments of the present disclosure. As shown in FIG. 11, during the process of the perfusion scanning, the density of contrast agent in the perfusion scanning site (e.g., a brain) may increase first and then decrease with time.

In some embodiments, the data removing module 960 may obtain an initial image reconstruction result at each of the plurality of time points by performing initial image reconstruction on the perfusion scanning data at the plurality of time points, respectively. The data removing module 960 may determine the time attenuation curve based on the image reconstruction result at each of the plurality of time points. More descriptions about the initial image reconstruction result and determining the time attenuation curve may refer to FIG. 3, FIG. 6, and the related descriptions.

In some embodiments, the reconstructed slice thickness of the initial image reconstruction is not less than 3 mm. For example, the reconstructed slice thickness of the initial image reconstruction may be 5mm or 6mm. The initial image reconstruction may be performed using a large slice thickness, which may be beneficial to obtain the initial image reconstruction result at each of the plurality of time points quickly.

In some embodiments, the data removing module 960 may identify the arterial region based on the initial image reconstruction results at the plurality of time points; and the time attenuation curve may be determined based on image data of the arterial region in the initial image reconstruction results at the plurality of time points.

In some embodiments, the data removing module 960 may establish the time attenuation curve based on the image data of the arterial region in the initial image reconstruction results at the plurality of time points only. In some embodiments, the data removing module 960 may identify the arterial region based on the initial image reconstruction results at the plurality of time points. The data removing module 960 may further establish a time attenuation curve of the arterial region based on the image data of the arterial region in the initial image reconstruction results at the plurality of time. The arterial region may include a region of the arterial location. In some embodiments, the arterial region may be a minimum region containing the arterial location. In some embodiments, the data removing module 960 may identify the arterial region of the initial image reconstruction result using a trained arterial region identification model (e.g., a machine learning model). In some embodiments, the data removing module 960 may identify the arterial region for an initial image reconstruction result at a time point (e.g., the first time point), and determine the position corresponding to the arterial region at the time point (e.g., a first time point) in other time points as the arterial region at other time points. Establishing the time attenuation curve based on the image data of the arterial region in the initial image reconstruction result can improve the establishing efficiency of the perfusion time attenuation curve.

In 830, the perfusion stage at the first time point may be determined based on the time attenuation curve.

In some embodiments, the data removing module 960 may determine the perfusion stage at the first time point based on a curve slope corresponding to the first time point on the time attenuation curve. The time attenuation curve may be a smoothed curve. In some embodiments, the data removing module 960 may determine the perfusion stage at the first time point according to an absolute value of a curve slope corresponding to the first time point on the time attenuation curve. For example, the absolute value of the curve slope corresponding to the first time point may be greater than the preset threshold (e.g., 1, 1.5, etc.), and the inflow and outflow stage at the first time point may be determined when the absolute value of the curve slope corresponding to the first time point is less than or equal to the preset threshold. In some embodiments, when the curve slope corresponding to the first time point is 0, it may be determined that the first time point is at the peak, that is to say, the first time point is in the arterial stage. According to the time attenuation curve shown in FIG. 11, in the arterial stage, the time attenuation curve may change rapidly and the absolute value of the slope may be large; and in the inflow and outflow stage, the time attenuation curve may change slowly and the absolute value of the slope is small. By determining the curve slope corresponding to the first time point based on the time attenuation curve, the perfusion stage at the first time point may be determined quickly and accurately.

In some embodiments, the data removing module 960 may determine a peak time based on the time attenuation curve, and further determine the perfusion stage at the first time point based on the interval between the first time point and the peak time. For example, the data removing module 960 may determine a corresponding time point of the peak time point as the peak time. In some embodiments, the data removing module 960 may determine the perfusion stage at the first time point according to the interval between the first time point and the peak time. For example, when the interval between the first time point and the peak time is less than or equal to 10 seconds, it may be determined that the first time point is in the arterial stage, and when the interval between the first time point and the peak time is greater than 10 seconds, it may be determined that the first time point is in the inflow and outflow stage. In some embodiments, the data removing module 960 may determine the perfusion stage at the first time point according to intervals prior to or after the interval between the first time point and the peak time. For example, the time point from 15 seconds prior to the peak time to 5 seconds after the peak time may be determined as being in the arterial stage, and the time point without the above range may be determined as being in the inflow and outflow stage. Such a flexible way of dividing the arterial stage is beneficial to adapt to a difference in the time it takes for the contrast agent to flow into the artery for each patient. By determining the perfusion stage at the first time point based on the interval between the first time point and the peak time, the determination process may be faster and the determination result may be more accurate.

In some embodiments, when the data removing module 960 determines the peak time, the image reconstruction module 920 may obtain a reconstruction result by performing the image reconstruction based on the perfusion scanning data at the peak time (or a peak phase); and the vascular analysis module 930 may perform the vascular analysis based on the reconstruction result.

In some embodiments, the image reconstruction module 920 may preprocess the perfusion scanning data of the peak phase; the image reconstruction module 920 may obtain the perfusion scanning image of the peak phase by reconstructing the preprocessed perfusion scanning data; and the image reconstruction module 920 may obtain a reconstruction result of the peak phase by postprocessing the perfusion scanning image of the peak phase. More descriptions about the image reconstruction may refer to FIG. 5 and the related descriptions.

In some embodiments, the vascular analysis module 930 may obtain a vascular analysis result by performing the vascular analysis automatically based on the reconstruction result of the peak phase. In some embodiments, the vascular analysis result may be used for a user (e.g., a doctor) to assess the vascular occlusion of the patient. In some embodiments, the vascular analysis module 930 may upload the vascular analysis result to an image archiving and communication system.

By incorporating the operation of vascular analysis in the process of perfusion analysis, it is beneficial to obtain the perfusion analysis result and vascular analysis result at the same time in a process of single data analysis, use the perfusion scanning data effectively and further be convenient for the doctor to make a more comprehensive judgment on the condition of the patient. In some embodiments, the system may fuse the perfusion analysis result and the vascular analysis result, to obtain a fusion analysis result for the vascular site of the patient.

In 840, whether the first time point is in the arterial stage may be determined. In some embodiments, the operation 840 may be performed by the data removing module 960.

When determining that the first time point is not in the arterial stage (e.g., in the inflow and outflow stage), the data removing module 960 may perform operation 850; and the perfusion scanning data at the first time point may remove. By removing the perfusion scanning at the first time point which does not in the arterial stage, the inaccurate of the perfusion scanning data at the first time point may be avoided to affect the accuracy of the overall calculation. After removing the perfusion scanning data at the first time point, the system 900 may be performed operation 860.

When determining that the first time point is in the arterial stage, the data removing module 960 may perform operation 870; and the perfusion scanning data at the first time point may retain. By not removing the perfusion scanning data at the first time point in the arterial stage, the excessive scanning interval in the arterial stage after removing the first time point in the arterial stage may cause serious data quality problems and further affect the accuracy of calculation results. After determining to retain the perfusion scanning at the first time point, the system 900 may perform operation 880 and/or operation 890.

In 860, the perfusion time attenuation curve and/or perfusion parameter may be obtained by performing the perfusion analysis based on the perfusion scanning data at the remaining time points that remove the first time point. In some embodiments, the operation 860 may be performed by the perfusion analysis module 970.

In 880, the perfusion time attenuation curve and/or perfusion parameter may be obtained by performing the perfusion analysis based on the perfusion scanning data at the plurality of time points. In some embodiments, the operation 880 may be performed by the perfusion analysis module 970. In some embodiments, when there is the first time point and the perfusion scanning data at the first time point retain, the perfusion analysis module 970 may perform the perfusion analysis on the perfusion scanning data at the plurality of time points obtained in the operation 710.

In 890, a user may be prompted for relevant information at the first time point by generating prompting information. In some embodiments, the operation 890 may be performed by the system 900 (e.g., the perfusion analysis module 970).

In some embodiments, the prompting information may include one or more of the following information: inform information at the first time point, time information at the first time point, the perfusion scanning information at the first time point, location information at the first time point in the time attenuation curve, information that the motion amplitude at the first time point is greater than the preset amplitude, information that leads to inaccurate results due to the existence of the first time point, and etc. In some embodiments, the display formats of the prompting information may include but are not limited to a voice prompt, an animation prompt, an image prompt, a text prompt, a pop-up prompt, or any combination thereof. The user for relevant information may be prompted at the first time point by the prompting information, which can make the user know that there is a relatively large movement range at the first time point, and further assist users to make correct analysis and judgment. In some embodiments, the system 900 may display the prompting information and optional operational information for the user. For example, the optional operational information may include deleting the first time point, reperforming the perfusion scanning, reperforming the perfusion analysis, etc.

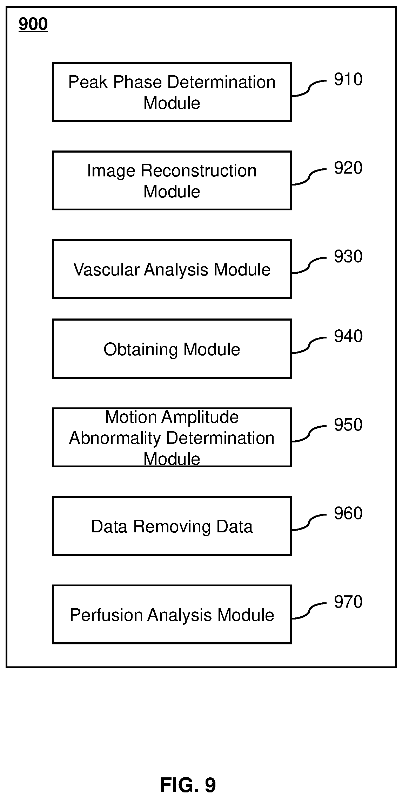

FIG. 9 is a block diagram illustrating an exemplary system 900 for data analysis based on perfusion scanning according to some embodiments of the present disclosure. In some embodiments, the system 900 may be performed by the device 1000 (e.g., the processor 1020). As shown in FIG. 9, the system 900 may include a peak phase determination module 910, an image reconstruction module 920, a vascular analysis module 930, an obtaining module 940, a motion amplitude abnormality determination module 950, a data removing module 960, and a perfusion analysis module 970. In some embodiments, the system 900 may be applied to the vascular analysis, the system 900 may also be referred to as a vascular analysis system. In some embodiments, the system 900 may be applied to the perfusion analysis, the system 900 may also be referred to as a perfusion analysis system.

The peak phase determination module 910 may be used to determine a peak phase. In some embodiments, the peak phase determination module 910 may be used to determine a peak phase in the plurality of phases based on the perfusion scanning data of the plurality of phases. In some embodiments, the peak phase determination module 910 may determine a time attenuation curve based on the initial reconstruction results of the plurality of phases. In some embodiments, the peak phase determination module 910 may determine the peak phase based on the time attenuation curve.

The image reconstruction module 920 may be used to perform the image reconstruction. In some embodiments, the image reconstruction 920 may be configured to obtain a reconstruction result by performing the image reconstruction based on the perfusion scanning data of the peak phase. In some embodiments, for the perfusion scanning data of each phase in the plurality of phases, the image reconstruction module 920 may obtain an initial image reconstruction result of each phase by performing the initial image reconstruction.

The vascular analysis module 930 may be used to perform the vascular analysis. In some embodiments, the vascular analysis module 930 may perform the vascular analysis based on the reconstruction result.

The obtaining module 940 may be used to obtain data and/or information in a process of obtaining the perfusion analysis. In some embodiments, the obtaining module 940 may be used to obtain the perfusion scanning data at the plurality of time points.

The motion amplitude abnormality determination module 950 may be used to whether a motion amplitude at the time point is abnormal. In some embodiments, the motion amplitude abnormality determination module 950 may determine, based on the perfusion scanning data, whether a motion amplitude at each of the plurality of time points is greater than a preset amplitude. In some embodiments, the motion amplitude abnormality determination module 950 may obtain variables of the perfusion scanning images at the time point and the perfusion scanning images at the adjacent time point, and determine whether the motion at each of the plurality of time points is greater than the preset amplitude based on the variables of the perfusion scanning images at the time point and the perfusion scanning images at the adjacent time points.

The data removing module 960 may be used to determine and remove the perfusion scanning data. In some embodiments, the data removing module 960 may determine whether to remove the perfusion scanning data at the first time point according to the perfusion stage at the first time point. In some embodiments, when the first time point is in the arterial stage, the data removing module 960 may retain the perfusion scanning data at the first time point. In some embodiments, when the first time point is in the inflow and outflow stage, the data removing module 960 may remove the perfusion scanning data at the first time point.

The perfusion analysis module 970 may be used to perform the perfusion analysis. In some embodiments, the perfusion analysis may be used to obtain the perfusion time attenuation curve and/or perfusion parameter by performing, based on the perfusion scanning data at remaining time points that remove the first time point, the perfusion analysis based on the perfusion scanning data at the plurality of time points, or the perfusion analysis.

It should be noted that the above description of the data analysis system based on the perfusion scanning data and modules is merely provided for the purposes of illustration, and not intended to limit the scope of the present disclosure. For persons having ordinary skills in the art, multiple variations and modifications may be made under the teachings of the present disclosure. In some embodiments, the peak phase determination module 910, the image reconstruction module 920, the data removing module 960, and the perfusion analysis module 970 may be different modules in a system, or implement the function of the above two or more modules by a module. For example, different modules may share a storage module, and different modules may have its own storage module. However, those variations and modifications do not depart from the scope of the present disclosure.

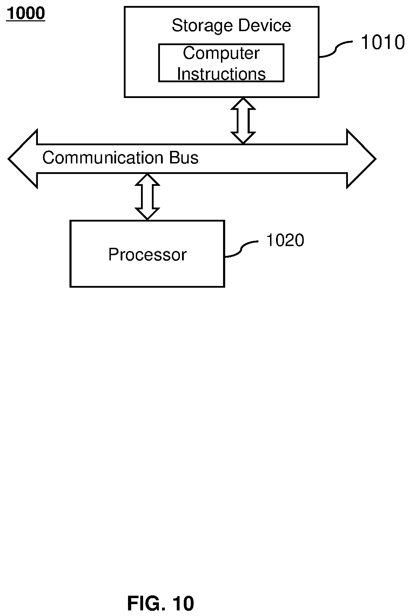

FIG. 10 is a structural diagram illustrating an exemplary device 1000 for data analysis based on perfusion scanning according to some embodiments of the present disclosure. As shown in FIG. 10, the device 1000 may include a storage device 1010, a processor 1020, and a communication bus. The storage device 1010 and the processor 1020 may implement a communication process by the communication bus. The processor 1020 may be used to perform the vascular analysis method and/or the perfusion analysis method provided in the above embodiments in the present disclosure. In some embodiments, the device 1000 may be applied to the vascular analysis, the device 1000 may also be referred to as a vascular analysis device. In some embodiments, the device 1000 may be applied to the perfusion analysis, the device 1000 may also be referred to as a perfusion analysis device.

In some embodiments, the processor 1020 may be implemented by a central processing unit, a server, a terminal device, or any other possible processing device. In some embodiments, the central processing unit, the server, the terminal device, or any other possible processing device may be implemented on a cloud platform. In some embodiments, the central processing unit, the server, or other processing devices may be interconnected with various terminal devices, and the terminal devices may complete the information processing work or part of the information processing work.

In some embodiments, the storage device 1010 (or a computer readable storage medium) may store data and/or instructions (e.g., computer instructions). In some embodiments, the storage device 1010 may store a preset scanning interval, a scanning time, a dose parameter, a postprocessing program, an image reconstruction program, etc. In some embodiments, the storage device 1010 may store computer instructions, and the processor 1020 (or the computer) may perform the vascular analysis method provided in any embodiments in the present discourse. In some embodiments, the storage device may include a mass storage, a removable storage, a volatile read-write memory, a read-only memory (ROM), etc., or any combination thereof. In some embodiments, the storage device may implement on a cloud platform.

The beneficial effects in the embodiments of the present discourse may include but are not limited to the followings: (1) an automated workflow that automatically identifies the peak phase and splits phase data for the vascular analysis may be provided; (2) the time attenuation curve may be determined by the initial reconstruction results of the plurality of phases, and the peak phase may be located automatically; a single-phase image reconstruction and the vascular analysis may be performed based on the perfusion scanning data of the peak phase, which may provide the user with vascular analysis results quickly and accurately and assist the user in making stroke diagnosis and treatment plans; (3) a one-stop automated analysis workflow for combined vascular analysis of cerebral perfusion can be improved, the reconstruction of useless data may be reduced, the reconstruction resources may be saved, the automatic efficiency may be improved, and the stroke DNT time may be shortened; (4) an automated perfusion analysis workflow may be provided to automatic evaluate the motion of the perfusion at each of the plurality time points, and whether to delete the time point may be determined automatically according to a stage of the rapid and outflow phase of the contrast agent at the time point; (5) the perfusion analysis method may perform automatic evaluation and analysis due to the patients and unqualified data quality control, which may give the user relatively more accurate results; (6) the perfusion analysis method can improve the processing workflow, reduce the reliance on user experience, and reduce the training cost; (7) the perfusion analysis method may reduce the rescanning situation caused by the limited user experience due to unqualified data to a certain extent, and reduce the patient dose. It should be noted that different embodiments may produce different beneficial effects. In different embodiments, the possible beneficial effects may be any one or combination of the above or any other possible beneficial effects.

The basic concepts have been described. Obviously, for those skilled in the art, the detailed disclosure may be only an example and may not constitute a limitation to the present disclosure. Although not explicitly stated here, those skilled in the art may make various modifications, improvements, and amendments to the present disclosure. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of the specification are not necessarily all referring to the same embodiment. In addition, some features, structures, or features in the present disclosure of one or more embodiments may be appropriately combined.

Moreover, unless otherwise specified in the claims, the sequence of the processing elements and sequences of the present application, the use of digital letters, or other names are not used to define the order of the application flow and methods. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various assemblies described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, this disclosure may not mean that the present disclosure object requires more features than the features mentioned in the claims. In fact, the features of the embodiments are less than all of the features of the individual embodiments disclosed above.

At last, it should be understood that the embodiments described in the disclosure are used only to illustrate the principles of the embodiments of this application. Other modifications may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for vascular analysis implemented on a machine including one or more processors and one or more storage devices, comprising:
determining (210) a peak phase in a plurality of phases based on perfusion scanning data of the plurality of phases, including:
for the perfusion scanning data of each phase in the plurality of phases, obtaining (310) an initial image reconstruction result of the phase by performing initial image reconstruction;
determining (320) a perfusion time attenuation curve based on the initial image reconstruction results of the plurality of phases; and
determining (330) the peak phase based on the perfusion time attenuation curve;
**characterised in that** the method further comprises
obtaining (220) a perfusion scanning image of the peak phase by performing, based on the perfusion scanning data of the peak phase, image reconstruction; and
performing (230) vascular analysis to assess the vascular occlusion of a patient based on the perfusion scanning image of the peak phase, including:
generating a preprocessed image by performing vascular analysis preprocessing on the reconstruction result;
performing vascular extraction on the preprocessed image; and
obtaining a vascular analysis result based on extracted vascular information.

2. The method of claim 1, wherein a reconstructed slice thickness of the initial image reconstruction is greater than the reconstructed slice thickness of the image reconstruction, the reconstructed slice thickness of the initial image reconstruction is not less than 3 mm, and the reconstructed slice thickness of the image reconstruction is less than 3 mm.

3. The method of any one claims of 1-2, wherein a count of reconstruction operations of the initial image reconstruction is less than a count of reconstruction operations of the image reconstruction.

4. The method of claim 3, wherein the reconstruction operations of the initial image reconstruction include:
obtaining (610) compressed perfusion scanning data;
preprocessing (620) the compressed perfusion scanning data; and
obtaining (640) a perfusion scanning image of each of the plurality of phases and a reconstruction result of the initial image reconstruction by reconstructing (630) the preprocessed compressed perfusion scanning data.

5. The method of claim 3 or 4, wherein the reconstruction operations of the image reconstruction include:
obtaining (510) the perfusion scanning data;
preprocessing (520) the perfusion scanning data; and
obtaining (550) a postprocessed reconstructed result by postprocessing (540) the perfusion scanning image of the peak phase.

6. The method of any one claims of 1-5, wherein the perfusion time attenuation curve is a perfusion time attenuation curve in an arterial region;
determining the perfusion time attenuation curve based on the initial image reconstruction results of the plurality of phases includes:
identifying the arterial region based on the initial image reconstruction results of the plurality of phases; and
determining the perfusion time attenuation curve based on image data of the arterial region in the initial image reconstruction results of the plurality of phases.

7. The method of any one claims of 1-6, wherein the perfusion scanning data is perfusion scanning data of a region of interest (ROI), the method further comprising:
obtaining (402) a reference image;
obtaining (404), based on the reference image, the ROI selected by a user; and
obtaining (406) the initial image reconstruction results of the plurality of phases of the ROI after performing perfusion scanning.

8. The method of any one claims of 1-7, wherein performing the vascular analysis based on the reconstruction result of the peak phase includes:
determining whether a motion amplitude of the peak phase is greater than a preset amplitude based on the reconstruction result of the peak phase;
in response to determining that the motion amplitude of the peak phase is less than or equal to the preset amplitude, performing the vascular analysis based on the reconstruction result of the peak phase; or
in response to determining that the motion amplitude of the peak phase is greater than the preset amplitude, prompting the user for relevant information of the motion amplitude of the peak phase by generating prompting information.

9. The method of claim 8, wherein in response to determining that the motion amplitude of the peak phase is greater than the preset amplitude, obtaining a reconstruction result of an adjacent phase of the peak phase by performing, based on the perfusion scanning data of the adjacent phase of the peak phase, image reconstruction; and
performing the vascular analysis based on the reconstruction result of the peak phase and the reconstruction result of the adjacent phase of the peak phase.

10. The method of claim 1, further comprising:
determining, based on the perfusion scanning data, whether a motion amplitude at each of the plurality of phases is greater than a preset amplitude; and
in response to determining that the motion amplitude at a first phase of the plurality of phases is greater than the preset amplitude, determining, based on a perfusion stage at the first phase, whether to remove the perfusion scanning data at the first phase.

11. The method of claim 10, wherein the perfusion scanning data at each of the plurality of phases includes a perfusion scanning image; and the determining, based on the perfusion scanning data, whether the motion amplitude at each of the plurality of phases is greater than the preset amplitude includes:
obtaining a variation between the perfusion scanning image at the each of the plurality of phases and the perfusion scanning image at an adjacent phase of the each of the plurality of phases; and
determining, based on the variation, whether the motion amplitude at the each of the plurality of phases is greater than the preset amplitude.

12. The method of claim 10 or claim 11, wherein the perfusion stage includes an arterial stage and an inflow and outflow stage; the method further comprising:
determining the perfusion stage at the first phase according to sampling intervals between the first phase and adjacent phases of the first phase, the sampling intervals between the first phase and the adjacent phases of the first phase including the sampling interval between the first phase and the adjacent phase prior to the first phase, and the sampling interval between the first phase and the adjacent phase after the first phase;
in response to determining that the sampling intervals between the first phase and the adjacent phases of the first phase are greater than a preset interval, determining that the first phase is in the inflow and outflow stage; or
in response to determining that at least one of the sampling intervals between the first phase and the adjacent phases of the first phase is not greater than the preset interval, determining that the first phase is in the arterial stage;
wherein the determining, based on the perfusion stage at the first phase, whether to remove the perfusion scanning data at the first phase includes:
in response to determining that the first phase is in the arterial stage, retaining the perfusion scanning data at the first phase; or
in response to determining that the first phase is in the inflow and outflow stage, removing the perfusion scanning data at the first phase.

13. The method of claim 10, further comprising:
obtaining the perfusion time attenuation curve or a perfusion parameter by performing, based on the perfusion scanning data at the plurality of phases or the perfusion scanning data at remaining phases that exclude the first phase, perfusion analysis.

14. A system for vascular analysis, comprising:
at least one storage device including a set of instructions; and
at least one processor in communication with the at least one storage device, wherein when executing the set of instructions, the at least one processor is directed to cause the device to perform operations including:
determining (210) a peak phase in a plurality of phases based on perfusion scanning data of the plurality of phases, including:
for the perfusion scanning data of each phase in the plurality of phases, obtaining (310) an initial image reconstruction result of the phase by performing initial image reconstruction;
determining (320) a perfusion time attenuation curve based on the initial image reconstruction results of the plurality of phases; and
determining (330) the peak phase based on the perfusion time attenuation curve;
**characterised in that** the processor is directed to cause the device to further perform
obtaining (220) a perfusion scanning image of the peak phase by performing, based on the perfusion scanning data of the peak phase, image reconstruction; and
performing (230) vascular to assess the vascular occlusion of a patient based on the perfusion scanning image of the peak phase, including:
generating a preprocessed image by performing vascular analysis preprocessing on the reconstruction result;
performing vascular extraction on the preprocessed image; and
obtaining a vascular analysis result based on extracted vascular information.

15. A non-transitory computer readable medium, comprising at least one set of instructions, wherein when executed by one or more processors of a computing device, the at least one set of instructions causes the computing device to perform a method, the method comprising:
determining (210) a peak phase in a plurality of phases based on perfusion scanning data of the plurality of phases, including:
for the perfusion scanning data of each phase in the plurality of phases, obtaining (310) an initial image reconstruction result of the phase by performing initial image reconstruction;
determining (320) a perfusion time attenuation curve based on the initial image reconstruction results of the plurality of phases; and
determining (330) the peak phase based on the perfusion time attenuation curve;
**characterised in that** the method further comprises
obtaining (220) a perfusion scanning image of the peak phase by performing, based on the perfusion scanning data of the peak phase, image reconstruction; and
performing (230) vascular analysis to assess the vascular occlusion of a patient based on the perfusion scanning image of the peak phase, including:
generating a preprocessed image by performing vascular analysis preprocessing on the reconstruction result;
performing vascular extraction on the preprocessed image; and
obtaining a vascular analysis result based on extracted vascular information.

## Patentansprüche

1. Verfahren zur Gefäßanalyse, das auf einer Maschine implementiert ist, die einen oder mehrere Prozessoren und eine oder mehrere Speichervorrichtungen einschließt, umfassend:
Bestimmen (210) einer Spitzenphase in einer Vielzahl von Phasen basierend auf Perfusionsscan-Daten der Vielzahl von Phasen, einschließlich:
für die Perfusionsscan-Daten jeder Phase in der Vielzahl von Phasen, Erhalten (310) eines anfänglichen Bildrekonstruktionsergebnisses der Phase durch Durchführen einer anfänglichen Bildrekonstruktion;
Bestimmen (320) einer Perfusionszeit-Dämpfungskurve basierend auf den anfänglichen Bildrekonstruktionsergebnissen der Vielzahl von Phasen; und
Bestimmen (330) der Spitzenphase basierend auf der Perfusionszeit-Dämpfungskurve;
**dadurch gekennzeichnet, dass** das Verfahren weiter umfasst
Erhalten (220) eines Perfusionsscan-Bildes der Spitzenphase durch Durchführen einer Bildrekonstruktion basierend auf den Perfusionsscan-Daten der Spitzenphase; und Durchführen (230) einer Gefäßanalyse zum Beurteilen der Gefäßokklusion eines Patienten basierend auf dem Perfusionsscan-Bild der Spitzenphase, einschließlich:
Erzeugen eines vorverarbeiteten Bildes durch Durchführen einer Gefäßanalyse-Vorverarbeitung an dem Rekonstruktionsergebnis;
Durchführen einer Gefäßextraktion auf dem vorverarbeiteten Bild;
und
Erhalten eines Gefäßanalyseergebnisses basierend auf extrahierten Gefäßinformationen.

2. Verfahren nach Anspruch 1, wobei eine rekonstruierte Schichtdicke der anfänglichen Bildrekonstruktion größer ist als die rekonstruierte Schichtdicke der Bildrekonstruktion, wobei die rekonstruierte Schichtdicke der anfänglichen Bildrekonstruktion nicht weniger als 3 mm beträgt und die rekonstruierte Schichtdicke der Bildrekonstruktion weniger als 3 mm beträgt.

3. Verfahren nach einem der Ansprüche 1-2, wobei eine Anzahl von Rekonstruktionsvorgängen der anfänglichen Bildrekonstruktion kleiner ist als eine Anzahl von Rekonstruktionsvorgängen der Bildrekonstruktion.

4. Verfahren nach Anspruch 3, wobei die Rekonstruktionsvorgänge der anfänglichen Bildrekonstruktion Folgendes einschließen:
Erhalten (610) komprimierter Perfusionsscan-Daten;
Vorverarbeiten (620) der komprimierten Perfusionsscan-Daten; und
Erhalten (640) eines Perfusionsscan-Bildes jeder der Vielzahl von Phasen und eines Rekonstruktionsergebnisses der anfänglichen Bildrekonstruktion durch Rekonstruieren (630) der vorverarbeiteten komprimierten Perfusionsscan-Daten.

5. Verfahren nach Anspruch 3 oder 4, wobei die Rekonstruktionsvorgänge der Bildrekonstruktion Folgendes einschließen:
Erhalten (510) der Perfusionsscan-Daten;
Vorverarbeiten (520) der Perfusionsscan-Daten;
Erhalten (550) eines nachbearbeiteten rekonstruierten Ergebnisses durch Nachbearbeiten (540) des Perfusionsscan-Bildes der Spitzenphase.

6. Verfahren nach einem der Ansprüche 1-5, wobei die Perfusionszeit-Dämpfungskurve eine Perfusionszeit-Dämpfungskurve in einem arteriellen Bereich ist;
wobei das Bestimmen der Perfusionszeit-Dämpfungskurve basierend auf den anfänglichen Bildrekonstruktionsergebnissen der Vielzahl von Phasen Folgendes einschließt:
Identifizieren des arteriellen Bereichs basierend auf den anfänglichen Bildrekonstruktionsergebnissen der Vielzahl von Phasen; und
Bestimmen der Perfusionszeit-Dämpfungskurve basierend auf Bilddaten des arteriellen Bereichs in den anfänglichen Bildrekonstruktionsergebnissen der Vielzahl von Phasen.

7. Verfahren nach einem der Ansprüche 1-6, wobei die Perfusionsscan-Daten Perfusionsscan-Daten eines Bereichs von Interesse (ROI) sind, wobei das Verfahren weiter umfasst:
Erhalten (402) eines Referenzbildes;
Erhalten (404) des von einem Benutzer ausgewählten ROI basierend auf dem Referenzbild; und
Erhalten (406) der anfänglichen Bildrekonstruktionsergebnisse der Vielzahl von Phasen des ROI nach Durchführen des Perfusionsscans.

8. Verfahren nach einem der Ansprüche 1-7, wobei Durchführen der Gefäßanalyse basierend auf dem Rekonstruktionsergebnis der Spitzenphase Folgendes einschließt:
Bestimmen, basierend auf dem Rekonstruktionsergebnis der Spitzenphase, ob eine Bewegungsamplitude der Spitzenphase größer als eine voreingestellte Amplitude ist;
als Reaktion auf Bestimmen, dass die Bewegungsamplitude der Spitzenphase kleiner oder gleich der voreingestellten Amplitude ist, Durchführen der Gefäßanalyse basierend auf dem Rekonstruktionsergebnis der Spitzenphase; oder
als Reaktion auf Bestimmen, dass die Bewegungsamplitude der Spitzenphase größer als die voreingestellte Amplitude ist, Auffordern des Benutzers zur Eingabe relevanter Informationen der Bewegungsamplitude der Spitzenphase durch Erzeugen von Aufforderungsinformationen.

9. Verfahren nach Anspruch 8, wobei als Reaktion auf Bestimmen, dass die Bewegungsamplitude der Spitzenphase größer als die voreingestellte Amplitude ist, ein Rekonstruktionsergebnis einer benachbarten Phase der Spitzenphase erhalten wird, indem basierend auf den Perfusionsscan-Daten der benachbarten Phase der Spitzenphase eine Bildrekonstruktion durchgeführt wird; und
Durchführen der Gefäßanalyse basierend auf dem Rekonstruktionsergebnis der Spitzenphase und dem Rekonstruktionsergebnis der benachbarten Phase der Spitzenphase.

10. Verfahren nach Anspruch 1, weiter umfassend:
Bestimmen, basierend auf den Perfusionsscan-Daten, ob eine Bewegungsamplitude in jeder der Vielzahl von Phasen größer als eine voreingestellte Amplitude ist; und
als Reaktion auf Bestimmen, dass die Bewegungsamplitude in einer ersten Phase der Vielzahl von Phasen größer als die voreingestellte Amplitude ist, Bestimmen, basierend auf einem Perfusionsstadium in der ersten Phase, ob die Perfusionsscan-Daten in der ersten Phase entfernt werden sollen.

11. Verfahren nach Anspruch 10, wobei die Perfusionsscan-Daten in jeder der Vielzahl von Phasen ein Perfusionsscan-Bild einschließen; und das Bestimmen, basierend auf den Perfusionsscan-Daten, ob die Bewegungsamplitude in jeder der Vielzahl von Phasen größer als die voreingestellte Amplitude ist, Folgendes einschließt:
Erhalten einer Variation zwischen dem Perfusionsscan-Bild in jeder der Vielzahl von Phasen und dem Perfusionsscan-Bild in einer benachbarten Phase jeder der Vielzahl von Phasen; und
Bestimmen, basierend auf der Variation, ob die Bewegungsamplitude in jeder der Vielzahl von Phasen größer als die voreingestellte Amplitude ist.

12. Verfahren nach Anspruch 10 oder Anspruch 11, wobei das Perfusionsstadium ein arterielles Stadium und ein Zufluss- und Abflusstadium einschließt; wobei das Verfahren weiter umfasst:
Bestimmen des Perfusionsstadiums in der ersten Phase entsprechend den Abtastintervallen zwischen der ersten Phase und benachbarten Phasen der ersten Phase, wobei die Abtastintervalle zwischen der ersten Phase und den benachbarten Phasen der ersten Phase das Abtastintervall zwischen der ersten Phase und der benachbarten Phase vor der ersten Phase und das Abtastintervall zwischen der ersten Phase und der benachbarten Phase nach der ersten Phase einschließen;
als Reaktion auf Bestimmen, dass die Abtastintervalle zwischen der ersten Phase und den benachbarten Phasen der ersten Phase größer als ein voreingestelltes Intervall sind, Bestimmen, dass die erste Phase in dem Zufluss- und Abflusstadium vorliegt; oder
als Reaktion auf Bestimmen, dass mindestens eines der Abtastintervalle zwischen der ersten Phase und den benachbarten Phasen der ersten Phase nicht größer als das voreingestellte Intervall ist, Bestimmen, dass die erste Phase in dem arteriellen Stadium vorliegt;
wobei das Bestimmen, basierend auf dem Perfusionsstadium in der ersten Phase, ob die Perfusionsscan-Daten bei der ersten Phase entfernt werden sollen, Folgendes einschließt:
als Reaktion auf Bestimmen, dass die erste Phase in dem arteriellen Stadium vorliegt, Beibehalten der Perfusionsscan-Daten in der ersten Phase; oder
als Reaktion auf Bestimmen, dass die erste Phase in dem Zufluss- und Abflussstadium vorliegt, Entfernen der Perfusionsscan-Daten in der ersten Phase.

13. Verfahren nach Anspruch 10, weiter umfassend:
Erhalten der Perfusionszeit-Dämpfungskurve oder eines Perfusionsparameters durch Durchführen einer Perfusionsanalyse basierend auf den Perfusionsscan-Daten der Vielzahl von Phasen oder der Perfusionsscan-Daten der verbleibenden Phasen, die die erste Phase ausschließen.

14. System zur Gefäßanalyse, umfassend:
mindestens eine Speichervorrichtung, die einen Satz von Anweisungen einschließt;
und
mindestens einen Prozessor in Kommunikation mit der mindestens einen Speichervorrichtung, wobei der mindestens eine Prozessor, wenn der Satz Anweisungen ausgeführt wird, angewiesen wird, die Vorrichtung zu veranlassen, Vorgänge durchzuführen, die Folgendes einschließen:
Bestimmen (210) einer Spitzenphase in einer Vielzahl von Phasen basierend auf Perfusionsscan-Daten der Vielzahl von Phasen, einschließlich:
für die Perfusionsscan-Daten jeder Phase in der Vielzahl von Phasen, Erhalten (310) eines anfänglichen Bildrekonstruktionsergebnisses der Phase durch Durchführen einer anfänglichen Bildrekonstruktion;
Bestimmen (320) einer Perfusionszeit-Dämpfungskurve basierend auf den anfänglichen Bildrekonstruktionsergebnissen der Vielzahl von Phasen; und
Bestimmen (330) der Spitzenphase basierend auf der Perfusionszeit-Dämpfungskurve;
**dadurch gekennzeichnet, dass** der Prozessor angewiesen wird, die Vorrichtung zu veranlassen, weiter durchzuführen
Erhalten (220) eines Perfusionsscan-Bildes der Spitzenphase durch Durchführen einer Bildrekonstruktion basierend auf den Perfusionsscan-Daten der Spitzenphase; und
Durchführen (230) einer vaskulären zum Beurteilen der Gefäßokklusion eines Patienten basierend auf dem Perfusionsscan-Bild der Spitzenphase, einschließlich:
Erzeugen eines vorverarbeiteten Bildes durch Durchführen einer Gefäßanalyse-Vorverarbeitung an dem Rekonstruktionsergebnis;
Durchführen einer Gefäßextraktion auf dem vorverarbeiteten Bild;
und
Erhalten eines Gefäßanalyseergebnisses basierend auf extrahierten Gefäßinformationen.

15. Nichtflüchtiges, computerlesbares Medium, das mindestens einen Satz von Anweisungen umfasst, wobei der mindestens eine Satz von Anweisungen, wenn von einem oder mehreren Prozessoren einer Rechenvorrichtung ausgeführt, die Rechenvorrichtung veranlasst, ein Verfahren durchzuführen, wobei das Verfahren umfasst:
Bestimmen (210) einer Spitzenphase in einer Vielzahl von Phasen basierend auf Perfusionsscan-Daten der Vielzahl von Phasen, einschließlich:
für die Perfusionsscan-Daten jeder Phase in der Vielzahl von Phasen, Erhalten (310) eines anfänglichen Bildrekonstruktionsergebnisses der Phase durch Durchführen einer anfänglichen Bildrekonstruktion;
Bestimmen (320) einer Perfusionszeit-Dämpfungskurve basierend auf den anfänglichen Bildrekonstruktionsergebnissen der Vielzahl von Phasen; und
Bestimmen (330) der Spitzenphase basierend auf der Perfusionszeit-Dämpfungskurve;
**dadurch gekennzeichnet, dass** das Verfahren weiter umfasst
Erhalten (220) eines Perfusionsscan-Bildes der Spitzenphase durch Durchführen einer Bildrekonstruktion basierend auf den Perfusionsscan-Daten der Spitzenphase; und
Durchführen (230) einer Gefäßanalyse zum Beurteilen des Gefäßokklusion eines Patienten basierend auf dem Perfusionsscan-Bild der Spitzenphase, einschließlich:
Erzeugen eines vorverarbeiteten Bildes durch Durchführen einer Gefäßanalyse-Vorverarbeitung an dem Rekonstruktionsergebnis;
Durchführen einer Gefäßextraktion auf dem vorverarbeiteten Bild;
und
Erhalten eines Gefäßanalyseergebnisses basierend auf extrahierten Gefäßinformationen.

## Revendications

1. Procédé d'analyse vasculaire mis en œuvre sur une machine incluant un ou plusieurs processeurs et un ou plusieurs dispositifs de stockage, comprenant :
la détermination (210) d'une phase de crête dans une pluralité de phases sur la base de données de balayage de perfusion de la pluralité de phases, incluant :
pour les données de balayage de perfusion de chaque phase dans la pluralité de phases, l'obtention (310) d'un résultat de reconstruction d'image initiale de la phase en réalisant une reconstruction d'image initiale ;
la détermination (320) d'une courbe d'atténuation de perfusion en fonction du temps sur la base des résultats de reconstruction d'image initiale de la pluralité de phases ; et
la détermination (330) de la phase de crête sur la base de la courbe d'atténuation de perfusion en fonction du temps ;
**caractérisé en ce que** le procédé comprend en outre
l'obtention (220) d'une image de balayage de perfusion de la phase de crête en réalisant, sur la base des données de balayage de perfusion de la phase de crête, une reconstruction d'image ; et la réalisation (230) d'une analyse vasculaire pour évaluer l'occlusion vasculaire d'un patient sur la base de l'image de balayage de perfusion de la phase de crête, incluant :
la génération d'une image prétraitée en réalisant un prétraitement d'analyse vasculaire sur le résultat de reconstruction ;
la réalisation d'une extraction vasculaire sur l'image prétraitée ; et
l'obtention d'un résultat d'analyse vasculaire sur la base d'informations vasculaires extraites.

2. Procédé selon la revendication 1, dans lequel une épaisseur de coupe reconstruite de la reconstruction d'image initiale est supérieure à l'épaisseur de coupe reconstruite de la reconstruction d'image, l'épaisseur de coupe reconstruite de la reconstruction d'image initiale n'est pas inférieure à 3 mm, et l'épaisseur de coupe reconstruite de la reconstruction d'image est inférieure à 3 mm.

3. Procédé selon l'une quelconque des revendications 1-2, dans lequel un nombre d'opérations de reconstruction de la reconstruction d'image initiale est inférieur à un nombre d'opérations de reconstruction de la reconstruction d'image.

4. Procédé selon la revendication 3, dans lequel les opérations de reconstruction de la reconstruction d'image initiale incluent :
l'obtention (610) de données de balayage de perfusion compressées ;
le prétraitement (620) des données de balayage de perfusion compressées ; et
l'obtention (640) d'une image de balayage de perfusion de chacune de la pluralité de phases et d'un résultat de reconstruction de la reconstruction d'image initiale en reconstruisant (630) les données de balayage de perfusion compressées prétraitées.

5. Procédé selon la revendication 3 ou 4, dans lequel les opérations de reconstruction de la reconstruction d'image incluent :
l'obtention (510) des données de balayage de perfusion ;
le prétraitement (520) des données de balayage de perfusion ; et
l'obtention (550) d'un résultat reconstruit post-traité par post-traitement (540) de l'image de balayage de perfusion de la phase de crête.

6. Procédé selon l'une quelconque des revendications 1-5, dans lequel la courbe d'atténuation de perfusion en fonction du temps est une courbe d'atténuation de perfusion en fonction du temps dans une région artérielle ;
la détermination de la courbe d'atténuation de perfusion en fonction du temps sur la base des résultats de reconstruction d'image initiale de la pluralité de phases inclut :
l'identification de la région artérielle sur la base des résultats de reconstruction d'image initiale de la pluralité de phases ; et
la détermination de la courbe d'atténuation de perfusion en fonction du temps sur la base de données d'image de la région artérielle dans les résultats de reconstruction d'image initiale de la pluralité de phases.

7. Procédé selon l'une quelconque des revendications 1-6, dans lequel les données de balayage de perfusion sont des données de balayage de perfusion d'une région d'intérêt (ROI), le procédé comprenant en outre :
l'obtention (402) d'une image de référence ;
l'obtention (404), sur la base de l'image de référence, de la ROI sélectionnée par un utilisateur ; et
l'obtention (406) des résultats de reconstruction d'image initiale de la pluralité de phases de la ROI après la réalisation d'un balayage de perfusion.

8. Procédé selon l'une quelconque des revendications 1-7, dans lequel la réalisation de l'analyse vasculaire sur la base du résultat de reconstruction de la phase de crête inclut :
la détermination permettant d'établir si une amplitude de mouvement de la phase de crête est supérieure à une amplitude prédéfinie sur la base du résultat de reconstruction de la phase de crête ;
en réponse à la détermination que l'amplitude de mouvement de la phase de crête est inférieure ou égale à l'amplitude prédéfinie, la réalisation de l'analyse vasculaire sur la base du résultat de reconstruction de la phase de crête ; ou
en réponse à la détermination que l'amplitude de mouvement de la phase de crête est supérieure à l'amplitude prédéfinie, la demande à l'utilisateur d'informations pertinentes en rapport avec l'amplitude de mouvement de la phase de crête en générant des informations d'invite.

9. Procédé selon la revendication 8, dans lequel, en réponse à la détermination que l'amplitude de mouvement de la phase de crête est supérieure à l'amplitude prédéfinie, l'obtention d'un résultat de reconstruction d'une phase adjacente de la phase de crête en réalisant, sur la base des données de balayage de perfusion de la phase adjacente de la phase de crête, une reconstruction d'image ; et
la réalisation de l'analyse vasculaire sur la base du résultat de reconstruction de la phase de crête et du résultat de reconstruction de la phase adjacente de la phase de crête.

10. Procédé selon la revendication 1, comprenant en outre :
la détermination, sur la base des données de balayage de perfusion, permettant d'établir si une amplitude de mouvement à chacune de la pluralité de phases est supérieure à une amplitude prédéfinie ; et
en réponse à la détermination que l'amplitude de mouvement à une première phase de la pluralité de phases est supérieure à l'amplitude prédéfinie, la détermination, sur la base d'une étape de perfusion à la première phase, permettant d'établir s'il convient de supprimer les données de balayage de perfusion à la première phase.

11. Procédé selon la revendication 10, dans lequel les données de balayage de perfusion à chacune de la pluralité de phases incluent une image de balayage de perfusion ; et la détermination, sur la base des données de balayage de perfusion, permettant d'établir si l'amplitude de mouvement à chacune de la pluralité de phases est supérieure à l'amplitude prédéfinie inclut :
l'obtention d'une variation entre l'image de balayage de perfusion à chacune de la pluralité de phases et l'image de balayage de perfusion à une phase adjacente de chacune de la pluralité de phases ; et
la détermination, sur la base de la variation, permettant d'établir si l'amplitude de mouvement à chacune de la pluralité de phases est supérieure à l'amplitude prédéfinie.

12. Procédé selon la revendication 10 ou la revendication 11, dans lequel l'étape de perfusion inclut une étape artérielle et une étape de flux entrant et de flux sortant ; le procédé comprenant en outre :
la détermination de l'étape de perfusion à la première phase conformément à des intervalles d'échantillonnage entre la première phase et des phases adjacentes de la première phase, les intervalles d'échantillonnage entre la première phase et les phases adjacentes de la première phase incluant l'intervalle d'échantillonnage entre la première phase et la phase adjacente avant la première phase, et l'intervalle d'échantillonnage entre la première phase et la phase adjacente après la première phase ;
en réponse à la détermination que les intervalles d'échantillonnage entre la première phase et les phases adjacentes de la première phase sont supérieurs à un intervalle prédéfini, la détermination que la première phase est à l'étape de flux entrant et de flux sortant ; ou
en réponse à la détermination qu'au moins un des intervalles d'échantillonnage entre la première phase et les phases adjacentes de la première phase n'est pas supérieur à l'intervalle prédéfini, la détermination que la première phase est à l'étape artérielle ;
dans lequel la détermination, sur la base de l'étape de perfusion à la première phase, permettant d'établir s'il convient de supprimer les données de balayage de perfusion à la première phase inclut :
en réponse à la détermination que la première phase est à l'étape artérielle, la conservation des données de balayage de perfusion à la première phase ; ou
en réponse à la détermination que la première phase est à l'étape de flux entrant et de flux sortant, la suppression des données de balayage de perfusion à la première phase.

13. Procédé selon la revendication 10, comprenant en outre :
l'obtention de la courbe d'atténuation de perfusion en fonction du temps ou d'un paramètre de perfusion en réalisant, sur la base des données de balayage de perfusion à la pluralité de phases ou des données de balayage de perfusion à des phases restantes qui excluent la première phase, une analyse de perfusion.

14. Système d'analyse vasculaire, comprenant :
au moins un dispositif de stockage incluant un ensemble d'instructions ; et
au moins un processeur en communication avec le au moins un dispositif de stockage, dans lequel, lors de l'exécution de l'ensemble d'instructions, le au moins un processeur est amené à conduire le dispositif à réaliser des opérations incluant :
la détermination (210) d'une phase de crête dans une pluralité de phases sur la base de données de balayage de perfusion de la pluralité de phases, incluant :
pour les données de balayage de perfusion de chaque phase dans la pluralité de phases, l'obtention (310) d'un résultat de reconstruction d'image initiale de la phase en réalisant une reconstruction d'image initiale ;
la détermination (320) d'une courbe d'atténuation de perfusion en fonction du temps sur la base des résultats de reconstruction d'image initiale de la pluralité de phases ; et
la détermination (330) de la phase de crête sur la base de la courbe d'atténuation de perfusion en fonction du temps ;
**caractérisé en ce que** le processeur est amené à conduire le dispositif à exécuter en outre
l'obtention (220) d'une image de balayage de perfusion de la phase de crête en réalisant, sur la base des données de balayage de perfusion de la phase de crête, une reconstruction d'image ; et
la réalisation (230) vasculaire pour évaluer l'occlusion vasculaire d'un patient sur la base de l'image de balayage de perfusion de la phase de crête, incluant :
la génération d'une image prétraitée en réalisant un prétraitement d'analyse vasculaire sur le résultat de reconstruction ;
la réalisation d'une extraction vasculaire sur l'image prétraitée ; et
l'obtention d'un résultat d'analyse vasculaire sur la base d'informations vasculaires extraites.

15. Support non transitoire lisible par ordinateur comprenant au moins un ensemble d'instructions, dans lequel, lorsqu'il est exécuté par un ou plusieurs processeurs d'un dispositif informatique, le au moins un ensemble d'instructions amène le dispositif informatique à réaliser un procédé, le procédé comprenant :
la détermination (210) d'une phase de crête dans une pluralité de phases sur la base de données de balayage de perfusion de la pluralité de phases, incluant :
pour les données de balayage de perfusion de chaque phase dans la pluralité de phases, l'obtention (310) d'un résultat de reconstruction d'image initiale de la phase en réalisant une reconstruction d'image initiale ;
la détermination (320) d'une courbe d'atténuation de perfusion en fonction du temps sur la base des résultats de reconstruction d'image initiale de la pluralité de phases ; et
la détermination (330) de la phase de crête sur la base de la courbe d'atténuation de perfusion en fonction du temps ;
**caractérisé en ce que** le procédé comprend en outre
l'obtention (220) d'une image de balayage de perfusion de la phase de crête en réalisant, sur la base des données de balayage de perfusion de la phase de crête, une reconstruction d'image ; et
la réalisation (230) d'une analyse vasculaire pour évaluer l'occlusion vasculaire d'un patient sur la base de l'image de balayage de perfusion de la phase de crête, incluant :
la génération d'une image prétraitée en réalisant un prétraitement d'analyse vasculaire sur le résultat de reconstruction ;
la réalisation d'une extraction vasculaire sur l'image prétraitée ; et
l'obtention d'un résultat d'analyse vasculaire sur la base d'informations vasculaires extraites.
